# EUROPEAN PATENT SPECIFICATION

(11) **EP 2 373 808 B1**
(45) Date of publication and mention of the grant of the patent: **25.11.2015**
(21) Application number: 09768222.3
(22) Date of filing: 07.12.2009
(51) Int. Cl.: C12Q 1/68

(54) **ENHANCED TAQMAN PROBE BASED AMPLIFICATION**
VERBESSERTE AMPLIFIKATION AUF TAQMAN-SONDENBASIS
AMPLIFICATION AMÉLIORÉE BASÉE SUR LES SONDES TAQMAN

(30) Priority: 09.12.2008 GB 0822406; 09.12.2008 GB 0822410; 05.02.2009 GB 0901847; 15.06.2009 GB 0910207
(43) Date of publication of application: 12.10.2011
(73) Proprietor: Oxitec Limited, Oxford, Oxfordshire OX14 4RX (GB)
(72) Inventor: FU, Guoliang, Oxfordshire OX14 4RX (GB)
(74) Representative: Webber, Philip Michael
(86) International application number: PCT/GB2009/002834
(87) International publication number: WO 2010/067055

(56) References cited:
- WO-A1-2006/035062
- US-A- 5 487 972
- US-B1- 7 319 022
- CAPLIN B E ET AL: "LightCycler hybridization probes, the most direct way to monitor PCR amplification for quantification and mutation detection", BIOCHEMICA, MANNHEIM, DE, no. 1, 1 January 1999 (1999-01-01), pages 5-8, XP002241778, ISSN: 0946-1310
- OLIVIER ET AL: "The Invader<(>R) assay for SNP genotyping", MUTATION RESEARCH, ELSEVIER, AMSTERDAM, NL, vol. 573, no. 1-2, 3 June 2005 (2005-06-03), pages 103-110, XP027632671, ISSN: 0027-5107 [retrieved on 2005-06-03]

## Description

Real-time PCR has been developed to quantify amplified products during PCR reactions. Real-time PCR is based on the principles that emission of fluorescence from dyes directly or indirectly associated with the formation of newly-synthesized amplicons or the annealing of primers with DNA templates can be detected and is proportional to the amount of amplicons in each PCR cycle. Real-time PCR is carried out in a closed-tube format and is quantitative. Several methods are currently available for performing real-time PCR, such as utilising TaqMan probes (U.S. Pat. Nos. 5,210,015 and 5,487,972, and Lee et al., Nucleic Acids Res. 21:3761-6, 1993), molecular beacons (U.S. Pat. Nos. 5,925,517 and 6,103,476, and Tyagi and Kramer, Nat. Biotechnol. 14:303-8, 1996), self-probing amplicons (scorpions) (U.S. Pat. No. 6,326,145, and Whitcombe et al., Nat. Biotechnol. 17:804-7, 1999), Amplisensor (Chen et al., Appl. Environ. Microbiol. 64:4210-6, 1998), Amplifluor (U.S. Pat. No. 6,117,635, and Nazarenko et al., Nucleic Acids Res. 25:2516-21, 1997, displacement hybridization probes (Li et al., Nucleic Acids Res. 30:E5, 2002), DzyNA-PCR (Todd et al., Clin. Chem. 46:625-30, 2000), fluorescent restriction enzyme detection (Cairns et al. Biochem. Biophys. Res. Commun. 318:684-90, 2004) and adjacent hybridization probes (U.S. Pat. No. 6,174,670 and Wittwer et al., Biotechniques 22:130-1, 134-8, 1997). Most of these probes consist of a pair of dyes (a reporter dye and an acceptor dye) that are involved in fluorescence resonance energy transfer (FRET), whereby the acceptor dye quenches the emission of the reporter dye. In general, the fluorescence-labeled probes increase the specificity of amplicon quantification. For example, US 7,319,022 describes methods for amplification and monitoring of oligonucleotide amplification in which a primer has an overlap with one or more bases of a detection probe, and wherein an oligonucleotide conjugate has a fluorophore and a quencher.

Another form of probe used in PCR is a double-stranded linear probe which has two complementary oligonucleotides (Morrison L. et al., Anal. Biochem., Vol. 183, pages 231-244 (1989); US 5,928,862). Double-stranded linear probes modified by shortening one of the two complementary oligonucleotides by a few bases to make a partially double-stranded linear probe, are also known in the art (Li et al., Nucleic Acids Research, Vol. 30, No. 2, e5 (2002)). US 2005/0227257 describes a slightly modified double stranded linear nucleic acid probe which is modified by shortening one of the two complementary oligonucleotides by more bases, compared to the above, to make a partially double-stranded linear probe.

U.S. Pat. Nos. 5,210,015 and 5,487,972 describe the 5' nuclease assay, also termed TaqMan assay. The TaqMan assay exploits the 5' nuclease activity of Taq DNA Polymerase to cleave a TaqMan probe during PCR. The TaqMan probe contains a reporter dye at the 5' end of the probe and a quencher dye at the 3' end of the probe. During the reaction, cleavage of the probe separates the reporter dye and the quencher dye, resulting in increased fluorescence of the reporter. Accumulation of PCR products is detected directly by monitoring the increase in fluorescence of the reporter dye. When the probe is intact, the close proximity of the reporter dye to the quencher dye results in suppression of the reporter fluorescence primarily by Förster-type energy transfer (Förster, 1948; Lakowicz, 1983). During PCR, if the target of interest is present, the probe specifically anneals between the forward and reverse primer sites. The 5' to 3' nucleolytic activity of the Taq DNA polymerase cleaves the probe between the reporter and the quencher only if the probe hybridizes to the target. The probe fragments are then displaced from the target, and polymerization of the strand continues. The 3' end of the probe is blocked to prevent extension of the probe during PCR. This process occurs in every cycle and does not interfere with the exponential accumulation of the product.

The general guideline for designing TaqMan probes and primers is as follows: design the primers as close as possible to, but without overlapping, the probe; the Tm of the probe should be about 10 degree higher than the Tm of the primers; select the strand that gives the probe more C than G bases; the five nucleotides at the 3' end of the primer should have no more than two G and/or C bases, and the reaction should be run on the two-step thermal profile with the annealing and extension under the same temperature of 60° C.

U.S. Pat. Nos. 5,210,015 and 5,487,972 also disclose that in the absence of primer extension, the primer and labeled oligonucleotide are adjacently bound to the target nucleic acid, such that the 5' nuclease of Taq polymerase cleaves the probe and sequential rounds of oligonucleotide annealing and cleavage of labeled fragments occur in a polymerization independent manner. The patent description also says that the "signal generated during PCR amplification could be augmented by this polymerization-independent activity". However, it is not clear and was not disclosed how this can be designed. One way to achieve this signal augmentation by the polymerization-independent cleavage, which may partially occur or may not happen, is to design the primer as close as possible to the probe. As the primer is adjacent to the probe, the cleavage may occur before the polymerization during the annealing/extension step of the PCR. However, this explanation is speculative as there is no hard experimental evidence.

There is a need, however, to improve the probe based real-time amplification assay. It would be desirable that the signal generation of the probe is more efficient than the current assay, with the cleavage of the probe being more precise, the design of assay more simple, and the reaction run under an optimal thermal profile. The present invention provides a solution about how to use the polymerization-independent cleavage of the polymerase to efficiently generate the signal during real time PCR.

To facilitate understanding of the invention, a number of terms are defined below.

As used herein, a "sample" refers to any substance containing or presumed to contain nucleic acids and includes a sample of tissue or fluid isolated from an individual or individuals.

As used herein, the terms "nucleic acid", "polynucleotide" and "oligonucleotide" refer to primers, probes, oligomer fragments to be detected, oligomer controls and unlabeled blocking oligomers and shall be generic to polydeoxyribonucleotides (containing 2-deoxy-D-ribose), polyribonucleotides (containing D-ribose), and any other type of polynucleotide which is an N-glycoside of a purine or pyrimidine base, or modified purine or pyrimidine bases. There is no intended distinction in length between the term "nucleic acid", "polynucleotide" and "oligonucleotide", and these terms will be used interchangeably. "Nucleic acid", "DNA" and similar terms also include nucleic acid analogs. The oligonucleotide is not necessarily physically derived from any existing or natural sequence but may be generated in any manner, including chemical synthesis, DNA replication, reverse transcription or a combination thereof.

When two different, non-overlapping or with some overlapping, oligonucleotides anneal to different regions of the same linear complementary nucleic acid sequence, and the 3' end of one oligonucleotide points toward the 5' end of the other, the former may be called the "upstream" oligonucleotide and the latter the "downstream" oligonucleotide.

As used herein, the terms "target sequence", "target nucleic acid", "target nucleic acid sequence" and "nucleic acids of interest" are used interchangeably and refer to a desired region which is to be either amplified, detected or both.

"Primer" as used herein refers to more than one primer and refers to an oligonucleotide, whether occurring naturally or produced synthetically, which is capable of acting as a point of initiation of synthesis when placed under conditions in which synthesis of a primer extension product which is complementary to a nucleic acid strand is induced i.e., in the presence of nucleotides and an agent for polymerization such as DNA polymerase and at a suitable temperature and buffer. Such conditions include the presence of four different deoxyribonucleoside triphosphates and a polymerization-inducing agent such as DNA polymerase or reverse transcriptase, in a suitable buffer ("buffer" includes substituents which are cofactors, or which affect pH, ionic strength, etc.), and at a suitable temperature. The primer is preferably single-stranded for maximum efficiency in amplification. The primers herein are selected to be substantially complementary to the different strands of each specific sequence to be amplified. This means that the primers must be sufficiently complementary to hybridize with their respective strands. A non-complementary nucleotide fragment may be attached to the 5'-end of the primer, with the remainder of the primer sequence being complementary to the diagnostic section of the target base sequence. Commonly, the primers are complementary, except when non-complementary nucleotides may be present at a predetermined primer terminus as described.

The complement of a nucleic acid sequence as used herein refers to an oligonucleotide which, when aligned with the nucleic acid sequence such that the 5' end of one sequence is paired with the 3' end of the other, is in "antiparallel association." Complementarity need not be perfect; stable duplexes may contain mismatched base pairs or unmatched bases.

Stability of a nucleic acid duplex is measured by the melting temperature, or "Tₘ." The Tₘ of a particular nucleic acid duplex under specified conditions, is the temperature at which half of the base pairs have disassociated.

The term "complementary to" is used herein in relation to a nucleotide that will base pair with another specific nucleotide. Thus adenosine is complementary to uridine or thymidine and guanosine is complementary to cytidine.

The term "identical" means that two nucleic acid sequences have the same sequence or a complementary sequence.

"Amplification" as used herein denotes the use of any amplification procedures to increase the concentration of a particular nucleic acid sequence within a mixture of nucleic acid sequences.

The term "label" as used herein refers to any atom or molecule which can be used to provide or aid to provide, a detectable (preferably quantifiable) signal, and can be attached to a nucleic acid or protein. Labels may provide signals detectable by fluorescence, radioactivity, colorimetry, gravimetry, magnetism, enzymatic activity and the like.

The term "adjacent" or "substantially adjacent" as used herein refers to the positioning of two oligonucleotides on its complementary strand of the template nucleic acid. The two oligonucleotides may be separated by 0 to about 40 nucleotides, more preferably, 0, 1, 2, 3, 4, 5, 6, 7, 8, 9 or 10 nucleotides. The zero nucleotide gap means that the two oligonucleotides directly abut one another. In other words, the two template regions hybridised by two oligonucleotides may be contiguous, i.e. there is no gap between the two template regions. Alternatively, the two template regions hybridised by the oligonucleotides may be separated by 1 to about 40 nucleotides.

The term "overlapping" as used herein refers to the positioning of two oligonucleotides on its complementary strand of the template nucleic acid. The two oligonucleotides may be overlapping by 1 to about 40 nucleotides, but preferably, about 1 to 10 nucleotides. In other words, the two template regions hybridised by oligonucleotides may have a common region which is complementary to both the oligonucleotides.

The terms "thermally cycling," "thermal cycling", "thermal cycles" or "thermal cycle" refer to repeated cycles of temperature changes from a total denaturing temperature, to an annealing (or hybridising) temperature, to an extension temperature and back to the total denaturing temperature. The terms also refer to repeated cycles of a denaturing temperature and an extension temperature, where the annealing and extension temperatures are combined into one temperature. A total denaturing temperature unwinds all double stranded fragments into single strands. An annealing temperature allows a primer to hybridize or anneal to the complementary sequence of a separated strand of a nucleic acid template. The extension temperature allows the synthesis of a nascent DNA strand of the amplicon. The term "single round of thermal cycling" means one round of denaturing temperature, annealing temperature and extension temperature. In the single round of thermal cycling, there may be internal repeat of annealing temperature and extension temperature. For example, a single round of thermal cycling may include a denaturing temperature, a annealing temperature, a extension temperature, another annealing temperature and another extension temperature.

The terms "reaction mixture", "amplification mixture" or "PCR mixture" as used herein refer to a mixture of components necessary to amplify at least one amplicon from nucleic acid templates. The mixture may comprise nucleotides (dNTPs), a thermostable polymerase, primers, and a plurality of nucleic acid templates. The mixture may further comprise a Tris buffer, a monovalent salt, and Mg²⁺. The concentration of each component is well known in the art and can be further optimized by an ordinary skilled artisan.

The terms "amplified product" or "amplicon" refer to a fragment of DNA amplified by a polymerase using a pair of primers in an amplification method such as PCR

As defined herein, "5' 3' nuclease activity" or "5' to 3' nuclease activity" or "5' nuclease activity" refers to that activity of a cleavage reaction including either a 5' 3' nuclease activity traditionally associated with some DNA polymerases, whereby nucleotides are removed from the 5' end of an oligonucleotide in a sequential manner, (i.e., E. coli DNA polymerase I has this activity whereas the Klenow fragment does not), or a 5' 3' endonuclease activity wherein cleavage occurs more than one phosphodiester bond (nucleotide) from the-5' end, or both, or a group of homologous 5'-3' exonucleases (also known as 5' nucleases) which trim the bifurcated molecules, the branched DNA structures produced during DNA replication, recombination and repair. In the present invention, such 5' nuclease is used for cleavage of the labeled oligonucleotide annealed to target nucleic acid sequence adjacent to the helper oligonucleotide.

The practice of the present invention will employ, unless otherwise indicated, conventional techniques of molecular biology, microbiology and recombinant DNA techniques, which are within the skill of the art.

In a first aspect, the invention provides a method for the detection or measurement of a target nucleic acid sequence in a sample, said process comprising:
(a) providing to an amplification assay containing said sample:
   (i) a pair of amplification primers,
      wherein the first amplification primer contains a sequence complementary to a region of one strand of the target nucleic acid and is capable of priming the synthesis of the complementary strand,
      and wherein the second primer contains a sequence complementary to a region of the second strand of the target nucleic acid and is capable of priming the synthesis of a strand which is complementary thereto,
      such that, upon amplification in the presence of the target nucleic acid, the stretch of target nucleic acid between the amplification primers is amplified;
   (ii) a helper oligonucleotide containing a sequence complementary to a first region of the target nucleic acid;
   (iii) a labeled oligonucleotide containing a sequence complementary to a second region of the target nucleic acid;
      wherein the helper oligonucleotide and the labeled oligonucleotide both anneal to the same strand of the target nucleic acid,
      wherein the second region is adjacent to 5' end of the first region,
      or partially including the first region, and
      wherein the helper oligonucleotide and the labeled oligonucleotide both anneal within the target nucleic acid region comprised between the binding regions of the amplification primers,
   wherein
   if the target nucleic acid is present in the sample, the helper oligonucleotide and the labeled oligonucleotide anneal to the target nucleic acid such that the 3' end of the helper oligonucleotide is adjacent to or overlaps the 5' end of the labeled oligonucleotide,
(b) carrying out an amplification reaction using a nucleic acid polymerase having 5' nuclease activity
   under conditions which would, if the target nucleic acid is present in the sample, be permissive for the steps of:
   (i) hybridising the amplification primers, helper oligonucleotide and labeled oligonucleotide to the target nucleic acid,
      wherein the 5' nuclease activity of the polymerase cleaves the annealed, labeled oligonucleotide, in a polymerisation independent manner, to release labeled fragments which are detectable; and
   (ii) extending the amplification primers, wherein the nucleic acid polymerase synthesizes primer extension products, and
(c) detecting and/or measuring the release of labeled fragments to determine the presence or absence or amount of the target nucleic acid sequence in the sample.

The extension condition may permit the 5' nuclease activity of the polymerases simultaneously releases labeled fragments from the annealed duplexes comprising labeled oligonucleotide and its complementary template nucleic acid sequences, thereby creating detectable labeled fragments.

The hybridisation and the extension conditions may be the same conditions. In other words, the hybridising and extending may occur in the same conditions.

The hybridisation and the extension conditions preferably are repeated at least once in a single round of thermal cycling, if the hybridisation and the extension conditions are not the same conditions, wherein said single round of thermal cycling comprises temperatures for denaturing, hybridising, extending, hybridising and extending. The traditional two steps or three steps of thermal cycling are extended here as comprising four or more steps of thermal cycling.

The 3' end of the helper oligonucleotide in the annealed duplex is preferably adjacent to the 5' end of an annealed, labeled oligonucleotide, having spacing effective to permit the cleavage of the labeled oligonucleotide in the absence of nucleic acid polymerisation, said spacing between the 3' end of the helper oligonucleotide in the annealed duplex and the 5' end of an annealed, labeled oligonucleotide is from 0 to 10 nucleotides.

Alternately, the 3' end of the helper oligonucleotide in the annealed duplex overlaps the 5' end of an annealed, labeled oligonucleotide, having overlapping region effective to permit the cleavage of the labeled oligonucleotide in the absence of nucleic acid polymerisation, wherein said overlapping region between the 3' end of the helper oligonucleotide in the annealed duplex and the 5' end of an annealed, labeled oligonucleotide has 1 to 15 nucleotides.

The 3' end of the helper oligonucleotide in the annealed duplex may have a single nucleotide at 3' terminus not complementary to the target nucleic acid sequence.

The labeled oligonucleotide comprises at least one label, preferably the labeled oligonucleotide comprises first and second labels wherein the first label is separated from the second label by a nuclease susceptible cleavage site.

The helper oligonucleotide is preferably not extended during amplification.

The labeled oligonucleotide can be a single-stranded double-dye labeled TaqMan probe, or is a molecular beacon probe, or is one of strands of a partially double stranded probe, wherein said partially double-stranded probe comprises first oligonucleotide which is capable of hybridising to second oligonucleotide, wherein each of first and second oligonucleotides comprises a member of interactive label pair.

In another aspect, the invention provides a method for monitoring nucleic acid amplification comprising:
(a) providing to an amplification assay containing said sample:
   (i) a pair of amplification primers,
      wherein the first amplification primer contains a sequence complementary to a region of one strand of the target nucleic acid and is capable of priming the synthesis of the complementary strand,
      and wherein the second primer contains a sequence complementary to a region of the second strand of the target nucleic acid and is capable of priming the synthesis of a strand which is complementary thereto,
      such that, upon amplification in the presence of the target nucleic acid, the stretch of target nucleic acid between the amplification primers is amplified;
   (ii) a set of multiple labeled oligonucleotides, wherein each of the labeled oligonucleotides anneals to the same strand of the target nucleic acid,
      wherein all the labeled oligonucleotides anneal within the target nucleic acid region comprised between the binding regions of the amplification primers,
   wherein
   if the target nucleic acid is present in the sample, the labeled oligonucleotides anneal to the target nucleic acid such that the 3' end of one of the labeled oligonucleotide is adjacent to or overlaps the 5' end of a downstream labeled oligonucleotide, one of the amplification primers is adjacent to or overlaps the 5' end of a downstream labeled oligonucleotide;
(b) carrying out an amplification reaction using a nucleic acid polymerase having 5' nuclease activity under conditions which would, if the target nucleic acid is present in the sample, be permissive for the steps of:
   (i) hybridising the amplification primers, labeled oligonucleotides to the target nucleic acid,
      wherein the annealed upstream labeled oligonucleotide acts as helper oligonucleotide, facilities the hybridisation of the downstream labeled oligonucleotide and/or enhances the cleavage of the annealed, labeled oligonucleotide by the 5' nuclease activity of the polymerase, in a polymerisation independent manner, to release labeled fragments which are detectable; and
   (ii) extending the amplification primers, wherein the nucleic acid polymerase synthesizes primer extension products,
      wherein the hybridisation condition and/or extension condition permits the 5' nuclease activity of the polymerase to cleave the annealed, labeled oligonucleotide and release labeled fragments; and
(c) detecting and/or measuring the release of labeled fragments to determine the presence or absence or amount of the target nucleic acid sequence in the sample.

In the step (b) (ii) extending the amplification primer, preferably the polymerase synthesizes a primer extension product while the 5' nuclease activity of the nucleic acid polymerases simultaneously releases labeled fragments from the annealed duplexes comprising labeled oligonucleotide and its complementary template nucleic acid sequences, thereby creating detectable labeled fragments. In other words, the method may be performed under extension conditions sufficient to promote nucleic acid polymerization, wherein the release of labeled fragments occurs during extension of the amplification primer.

Also disclosed herein is a method for the detection or measurement of a target nucleic acid sequence in a sample, said process comprising:
(a) contacting a sample comprising single-stranded nucleic acids with an first oligonucleotide primer containing a sequence complementary to a first region of the target nucleic acid and a labeled oligonucleotide containing a sequence complementary to a second region of the same target nucleic acid sequence strand, overlapping the first region defined by the first oligonucleotide primer, to create a mixture of duplexes during hybridization conditions, if the target nucleic acid sequence is present in the sample, wherein the duplexes comprise the target nucleic acid annealed to the first oligonucleotide primer and to the labeled oligonucleotide such that the 3' end of the first oligonucleotide primer overlaps the 5' end of the labeled oligonucleotide;
(b) maintaining the mixture of step (a) with a template-dependent nucleic acid polymerase having a 5' to 3' nuclease activity under conditions sufficient to permit the 5' to 3' nuclease activity of the polymerase to cleave the annealed, labeled oligonucleotide and release labeled fragments; and
(c) detecting and/or measuring the release of labeled fragments and thereby detecting and/or measuring the target nucleic acid in the sample.
wherein the 3' end of the first oligonucleotide primer in the annealed duplex of step (a) overlaps the 5' end of an annealed, labeled oligonucleotide, having overlapping length effective to permit the release of labeled fragments in the absence of nucleic acid polymerisation.

The method may be performed under extension conditions sufficient to promote nucleic acid polymerization, wherein the release of labeled fragments occurs during extension of the first oligonucleotide primer.

Any above mentioned amplification is preferably a polymerase chain reaction (PCR), wherein the hybridisation and the extension conditions are repeated at least once in a single round of thermal cycling reaction if the hybridisation and the extension conditions are not the same conditions, wherein said single round of thermal cycling reaction comprises temperatures for denaturing, hybridising, extending, hybridising and extending.

Alternatively, the amplification may be an isothermal amplification.

Also disclosed herein is a kit for detecting a nucleic acid sequence in a sample comprising:
(a) a set of oligonucleotide primers, wherein a first primer contains a sequence complementary to a region in one strand of the nucleic acid sequence and primes the synthesis of a first extension product, and a second primer contains a sequence complementary to a region in said first extension product and primes the synthesis of a nucleic acid strand complementary to said first extension product, such that, upon amplification in the presence of the target nucleic acid, the stretch of target nucleic acid between the amplification primers is amplified;
(b) at least one helper oligonucleotide containing a sequence complementary to a region of the target nucleic acid and at least one labeled oligonucleotide containing a sequence complementary to a second region of the same target nucleic acid sequence strand, but not including or partially including the nucleic acid defined by the helper oligonucleotide, wherein said helper oligonucleotide and said labeled oligonucleotide are capable of hybridising to the target sequence to create duplexes during hybridisation conditions, wherein the duplexes comprise the target nucleic acid annealed to the helper oligonucleotide and to the labeled oligonucleotide such that the 3' end of the helper oligonucleotide is adjacent to or overlaps with the 5' end of the labeled oligonucleotide, wherein said helper oligonucleotide and said labeled oligonucleotide anneals within the target nucleic acid region comprised between the binding regions of the amplification primers of part (a), wherein said labeled oligonucleotide is blocked at the 3' terminus to prohibit incorporation of said labeled oligonucleotide into a primer extension product,
wherein said blocking is achieved by adding a chemical moiety to the 3' hydroxyl of the last nucleotide,
wherein said labeled oligonucleotide and said helper oligonucleotide anneal within the nucleic acid sequence concurrently with each oligonucleotide primer priming the synthesis of the first extension product or the nucleic acid strand complementary to the first extension product.

The inventor has found that the helper oligonucleotide greatly enhances the fluorescence signal of the real-time PCR reaction, resulting in strong signal and early Ct. The principle behind this phenomenon is likely that the 5' nuclease activity of the polymerase cleaves the annealed, labeled oligonucleotide which is hybridised to the target sequence adjacent to the helper oligonucleotide, in a polymerization independent manner, to release labeled fragments which are detectable. However, the inventor does not want to limit the theory for the above; another principle may be responsible. For example, the simple hybridisation of the helper oligonucleotide may enhance the hybridisation of the labeled oligonucleotide, which may not involve cleavage of the nuclease activity of a polymerase. Nevertheless, if the cleavage does occur, it is advantageous that the cleavage occurs in the polymerisation independent manner. Alternatively, the method is performed under conditions sufficient to promote nucleic acid polymerization, wherein the release of labeled fragments occurs during extension of the oligonucleotide primer.

The amplification reaction can be any amplification method, such as PCR, SDA, NASBA, LAMP, 3SR, ICAN, TMA, Helicase-dependent isothermal DNA amplification and the like. PCR is a preferred amplification method.

The amplification reaction mixture will comprise standard amplification reagents. Amplification reagents can conveniently be classified into four classes of components: (i) an aqueous buffer, often including, without limitation a magnesium salt, (ii) amplification substrates, such as DNA or RNA, (iii) one or more oligonucleotide primers (normally two primers for each target sequence, the sequences defining the 5' ends of the two complementary strands of the double-stranded target sequence when PCR is employed), and (iv) an amplification enzyme such as a polynucleotide polymerase (for example, Taq polymerase for PCR or RNA polymerase for TMA), or a ligase. Appropriate nucleoside triphosphates will also generally be required. Additional reagents or additives can also be included at the discretion of the skilled artisan and selection of these reagents is within the skill of the ordinary artisan. Of course, when the amplification reagents are used to cause both reverse transcription and amplification, the reverse transcription reagents are also included in the amplification reagents. Selection of amplification reagents, according to the method of amplification reaction used, is within the skill of the ordinary artisan.

The various aspects of the invention are based on polymerase with 5' nuclease activity in an amplification reaction. The 5' nuclease activity of a polymerase cleaves mononucleotides or small oligonucleotides from an oligonucleotide annealed to its larger, complementary polynucleotide. In order for cleavage to occur efficiently, an upstream helper oligonucleotide may be annealed to the same larger polynucleotide.

The helper oligonucleotide and labeled oligonucleotide can be designed such that they anneal in close proximity, or overlap, on the complementary target nucleic acid; the 5' nuclease of a polymerase cleaves the annealed, labeled oligonucleotide to release the labeled fragment. This process does not require polymerization. Alternatively, polymerisation may occur before the 5' nuclease cleaves the labeled oligonucleotide. As the polymerisation continues, the polymerase with 5' nuclease activity progressively cleaves the helper oligonucleotide and labeled oligonucleotide. This cleaving continues until the remainder of the oligonucleotide has been destabilized, to the extent that it dissociates from the template molecule. It is preferred that the polymerisation-independent cleavage occurs before the polymerisation reaches the annealed, labeled oligonucleotide.

The inventor has also found that repeat of hybridisation and extension conditions in a single round of thermal cycle reaction greatly enhance signal generation. This may be due to two or more cleavage events occurring in a single round of thermal cycle reaction. The single round of thermal cycling reaction may comprise temperatures for denaturing, hybridising, extending, hybridising and extending.

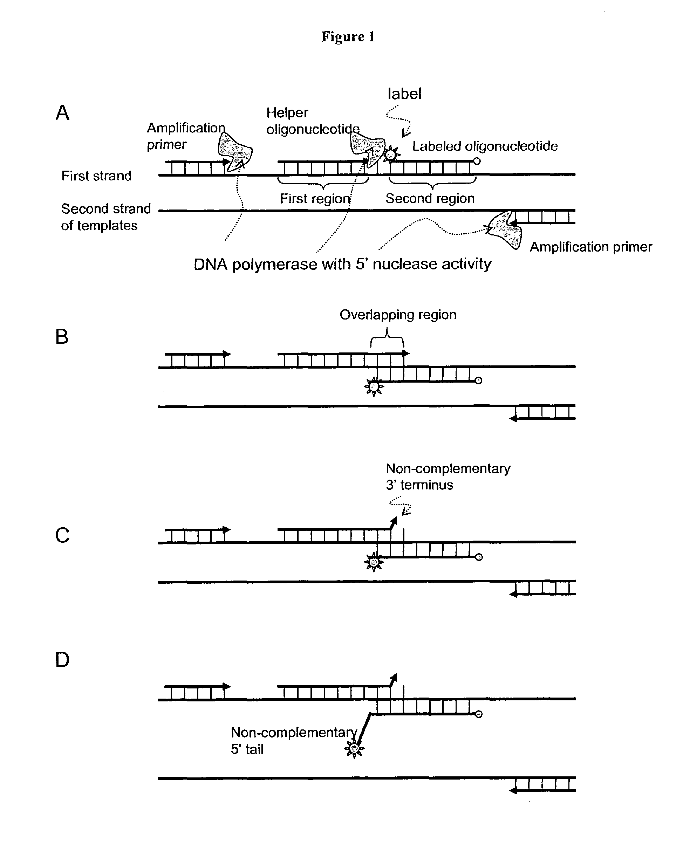

In one embodiment, the 3' end of the helper oligonucleotide in the annealed duplex with the template is adjacent to the 5' end of an annealed, labeled oligonucleotide. The spacing between the 3' end of the helper oligonucleotide and the 5' end of the labeled oligonucloetide is effective to permit the cleavage of the labeled oligonucleotide in the absence of nucleic acid polymerisation (Fig. 1A). Being annealed in this way, the labeled oligonucleotide can be cleaved by the 5' nuclease of the polymerase. The spacing between the 3' end of the helper oligonucleotide in the annealed duplex and the 5' end of an annealed, labeled oligonucleotide can be from 0 to 20 nucleotides, preferably from 0 to 10 nucleotides.

In another embodiment, the 3' end of the helper oligonucleotide in the annealed duplex overlaps with the 5' end of an annealed, labeled oligonucleotide (Fig. 1B), wherein the 3' end of the helper oligonucleotide and the 5' end of an annealed, labeled oligonucleotide comprise sequences complementary to the same portion of the target sequence, therefore, they are capable of hybridising to a stretch of the same sequence in the target nucleic acid, having overlapping length effective to permit the cleavage of the labeled oligonucleotide in the absence of nucleic acid polymerisation. The overlapping space between the 3' end of the helper oligonucleotide in the annealed duplex and the 5' end of an annealed, labeled oligonucleotide can be any length as long as both oligonucleotides are capable of binding to the target nucleic acid stably and being cleaved by the 5' nuclease of the polymerase. However, a length from 1 to 15 nucleotides is preferable.

In a further embodiment, the 3' end of the helper oligonucleotide in the annealed duplex has a single nucleotide at 3' terminus not complementary to the target nucleic acid sequence (Fig. 1C). To be able to facilitate the cleavage of the adjacent labeled oligonucleotide, the 3' terminus oligonucleotide can be complementary to the target sequence, but this is not necessary. It has been demonstrated that the 3' terminal nucleotide not complementary to the target sequence can also support the cleavage of the labeled oligonucleotide. It is preferred that the helper oligonucleotide is not extended during amplification. Non-extending of the helper oligonucleotide can be achieved either by the non-complementary 3' terminus nucleotide, or being blocked by a blocking moiety. The helper oligonucleotide may, or may not comprise a label. It normally does not comprise a label.

For the best performance of the present invention, the helper oligonucleotide may have a higher Tm (melting temperature) than the Tm of the labeled oligonucleotide when hybridised with the target nucleic acid, although this requirement is not essential. It is also desirable that the helper oligonucleotide has a higher Tm (melting temperature) than the Tm of the amplification primer when hybridised with the target nucleic acid.

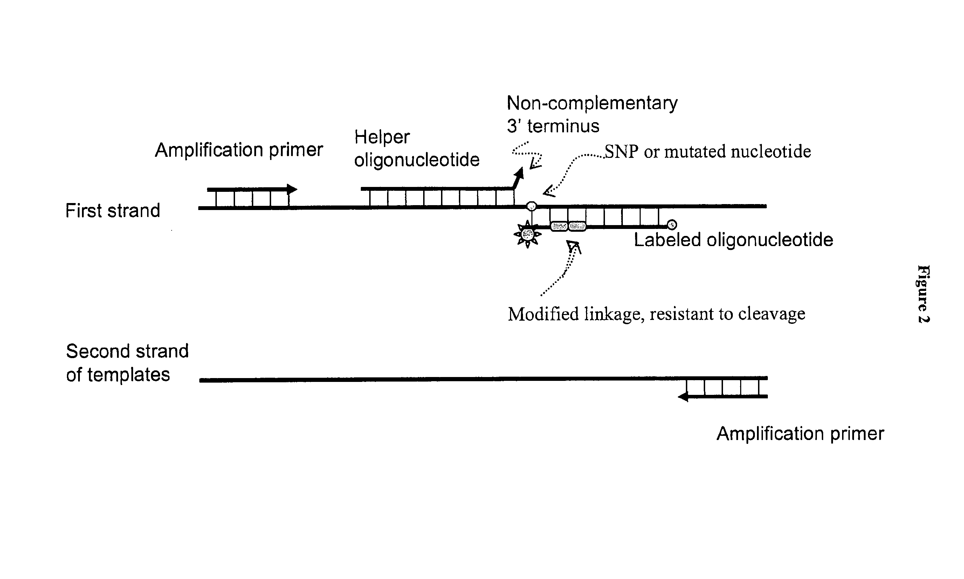

The nucleotide(s) within the labeled oligonucleotide may be modified to control nuclease cleavage specificity. It may be designed so that one particular nucleotide is sensitive to being cleaved, whereas other nucleotides cannot be cleaved. In particular, the nucleotide sensitive to being cleaved may be the nucleotide matching the SNP or the mutated nucleotide in the target nucleic acids (Fig. 2)

The labeled oligonucleotide comprises at least one label. It is desirable that the labeled oligonucleotide comprises first and second labels, wherein the first label is separated from the second label by a nuclease susceptible cleavage site. The labeled oligonucleotide may be labeled at the 5' terminus. The labeled oligonucleotide may further comprise tail(s) of non-nucleic acids or a sequence of nucleotides which are non-complementary to the target nucleic acid sequence (Fig. 1D). The label may be attached to a nucleotide in the tail or non-complementary sequence. It is preferred that the label is at the 5' terminus and is separated from the sequence complementary to the target nucleic acid sequence by the tail or non-complementary sequence. Alternatively, the label may be attached to an internal sequence of the oligonucleotide.

It is preferred that the labeled oligonucleotide comprises a pair of interactive signal-generating labels effectively positioned on the oligonucleotide to quench the generation of a detectable signal, said labels being separated by a site within the oligonucleotide susceptible to nuclease cleavage, thereby allowing the nuclease activity of the nucleic acid polymerase to separate the first interactive signal-generating label from the second interactive signal-generating label by cleaving at the susceptible site, thereby yielding a detectable signal. Normally, the labeled oligonucleotide has a blocked 3' terminus to prevent extension by the nucleic acid polymerase.

It is preferred that the cleavage occurs before the polymerisation reaches the labeled oligonucleotide; alternatively, the cleavage of the labeled oligonucleotide may occur during extension of the amplification primer.

The helper oligonucleotide comprises a sequence complementary to a first region of the target nucleic acid, while the labeled oligonucleotide comprises a sequence complementary to a second region of the same target nucleic acid sequence strand. The first region and the second region of the target sequence may not include each other, for example, the distance between the first region and second region may be 0 to 40 nucleotides, or preferably 0 to 10 nucleotides. Alternatively, the first region and second region may partially include each other. The first region and second region may have 1 to 10 nucleotide of overlapping sequence.

The helper oligonucleotide may be designed not to be extended. The helper oligonucleotide may have a 3' terminus nucleotide not complementary to the target sequence, so that it is not extended during the amplification. Alternatively, the 3' terminus nucleotide of the helper oligonucleotide may be modified so that it is not extendable by a polymerase. Some modifications of the 3' terminus of the helper oligonucleotide do not affect the efficiency of the cleavage, while other modifications may reduce the activity of the cleavage. The helper oligonucleotide modified with 3' phosphate enhances the signal generation. Alternatively, the helper oligonucleotide may be designed to be extendable as a normal primer.

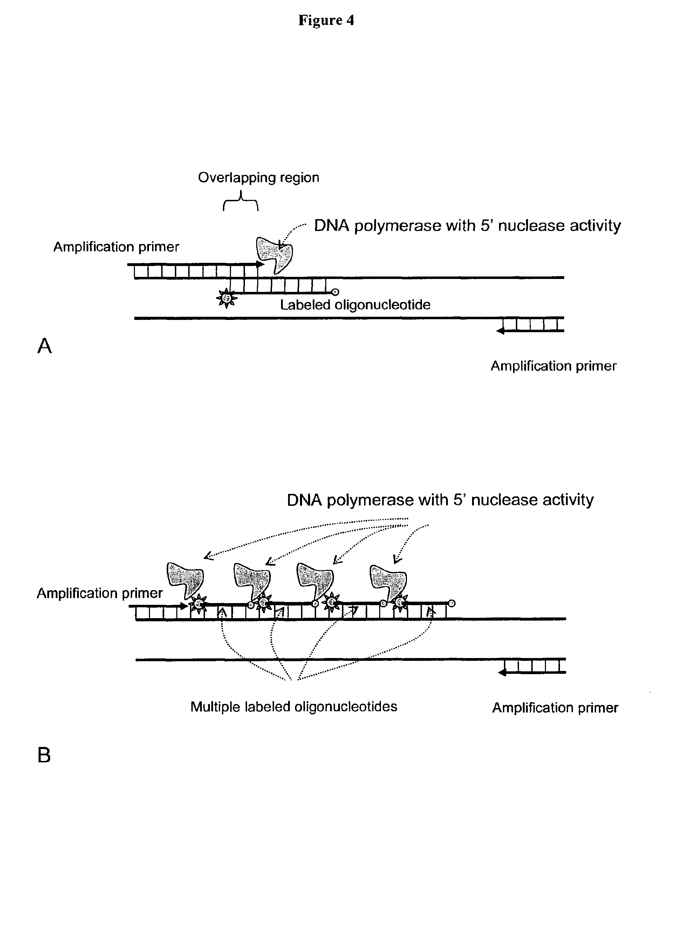

The helper oligonucleotide is preferably not labeled. In another embodiment of the present invention, the helper oligonucleotide is labeled. In fact, the method of present invention can comprise multiple labeled oligonucleotides (Fig. 4B). Some labeled oligonucleotides are actually functioning as helper oligonucleotide. When the multiple labeled oligonucleotides simultaneously anneal to the template nucleic acid next to each other, the 5' nuclease of the polymerase cleave each labeled oligonucleotides in a polymerisation-independent manner. The multiple labeled oligonucleotides annealed to the template can be adjacent to each other, or overlap. The gap or overlapping space can be the same as described above regarding the relationship between the helper oligonucleotide and labeled oligonucleotide.

The labeled oligonucleotide may not comprise a 5' non-complementary tail. Alternatively, the labeled oligonucleotide may comprise 5' nucleotides or non-naturally occurring nucleotide or chemical link tail. In the present invention, at least one label is attached to the labeled oligonucleotide (also referred to as a probe). The label may be attached to the 5' end of the 5' non-complementary tail or internally in the tail. Thus, the labeled fragment cleaved by the 5' nuclease activity of the polymerase can be detected. Subsequently, any of several strategies may be employed to distinguish the uncleaved labeled oligonucleotide from the cleaved fragments thereof. In this manner, the present invention permits identification of those nucleic acid samples which contain sequences complementary to the helper and labeled oligonucleotides.

In the present invention, the probe can be in any form. For the first instance, the probe can be in the single-stranded linear form like the TaqMan probe, or the stem-loop probe (like the molecular beacon). For the second instance, the probe may comprise a double stranded portion formed by two oligonucleotides. One example of such a probe may comprise: a first oligonucleotide which comprises a first region substantially complementary to part of one target nucleic acid and a second region, and at least one second oligonucleotide which comprises a region substantially complementary to the second region of the first oligonucleotide, such that the first and second oligonucleotides are capable of forming a double-stranded portion. The 5' end of one of the oligonucleotides is labeled with a fluorophore and the 3' end of the other oligonucleotide is labeled with a quencher. In the presence of a target, the first oligonucleotide anneals to the target, while the second oligonucleotide is displaced by the target. As a result, the first oligonucleotide (the probe) anneals to the target nucleic acid adjacent to the helper oligonucleotide and is cleaved, therefore becoming substantially more fluorescent.

As is known in the prior art, a double-labeled single stranded oligonucleotide can form a random-coiled structure when it is in single-stranded form and at certain permissive temperatures. This kind of linear oligonucleotide probe in solution behaves like a random coil: its two ends occasionally come close to one another, resulting in a measurable change in energy transfer. In the present invention, such a probe hybridises to the target nucleic acid adjacent to the helper oligonucleotide and is cleaved by a polymerase. The cleavage of the probe forces the two ends of the probe apart, disrupting the interaction between the two labels, and thus causing a fluorescence emission change.

A double-labeled oligonucleotide can also form a stem-loop structure known such as a molecular beacon. Molecular beacon probes are single-stranded oligonucleic acid probes that can form a hairpin structure, in which a fluorophore and a quencher are usually placed on the opposite ends of the oligonucleotide. At either end of the probe short complementary sequences allow the formation of an intramolecular stem, which enables the fluorophore and the quencher to come into close proximity. The loop portion of the molecular beacon is complementary to a target nucleic acid of interest. Binding of this probe to its target nucleic acid of interest forms a hybrid that forces the stem apart. This causes a conformation change that moves the fluorophore and the quencher away from each other and leads to a more intense fluorescent signal(Tyagi S. and Kramer F. R., Nature Biotechnology, Vol. 14, pages 303-308 (1996)). In the present invention, we provide an enhanced alternative signal generation strategy for the molecular beacon probe. The probe hybridises to the target nucleic acid adjacent to the helper oligonucleotide and is cleaved by a polymerase. The cleavage of the probe forces the two ends of the probe apart, disrupting the interaction between the two labels, and thus causing a fluorescence emission change. Therefore, the signal generated in this strategy, through the cleavage of the probe is different from the traditional signal generation by hybridisation of the probe to the target sequence.

Each probe comprises a detectable label which is capable of producing a changeable signal. The label on the probe may be a fluorophore, or the probe may comprise an interactive pair of labels, for example fluorophores and/or non-fluorophore dyes. One example of such interactive labels is a fluorophore-quencher pair. The label on the probe can be located anywhere, as long as it interacts with other labels or other entities such as G nucleotides on the oligonucleotide.

Typically, the fluorophore and the quencher are attached to the probes, such that when the probe is bound to a template sequence and is cleaved, the fluorophore and the quencher are separated.

"Fluorophore" is used herein to refer to a moiety that absorbs light energy at a defined excitation wavelength and emits light energy at a different defined wavelength.

As used herein, the term "quencher" includes any moiety that is capable of absorbing the energy of an excited fluorescent label when it is located in close proximity to the fluorescent label and is capable of dissipating that energy. A quencher can be a fluorescent quencher or a non-fluorescent quencher, which is also referred to as a dark quencher. The fluorophores listed above can play a quencher role if brought into proximity to another fluorophore, wherein either FRET quenching or contact quenching can occur. It is preferred that a dark quencher which does not emit any visible light is used. Examples of dark quenchers include, but are not limited to, DABCYL (4-(4'-dimethylaminophenylazo) benzoic acid) succinimidyl ester, diarylrhodamine carboxylic acid, succinimidyl ester (QSY-7), and 4',5'-dinitrofluorescein carboxylic acid, succinimidyl ester (QSY-33), quencherl, or "Black hole quenchers" (BHQ-1, BHQ-2 and BHQ-3), nucleotide analogs, nucleotide G residues, nanoparticles, and gold particles.

The present invention permits the detection of the target nucleic acid sequences during amplification of this target nucleic acid. In the present invention, a labeled oligonucleotide and a helper oligonucleotide are added concomitantly with the primers at the start of a PCR. The signal generated by the cleavage of the labeled nucleotide(s) of the probe, provides a means for detection of the target sequence during its amplification. If the target sequence is present, the helper oligonucleotide and the labeled oligonucleotide anneal to the target nucleic acid sequence. The duplexes comprise the target nucleic acid annealed to both the helper oligonucleotide and the labeled oligonucleotide such that the 3' end of the helper oligonucleotide is adjacent to or overlaps with the 5' end of the labeled oligonucleotide, wherein said helper oligonucleotide and said labeled oligonucleotide anneal within the target nucleic acid region comprised between the binding regions of the amplification primers. Upon the hybridisation of the helper oligonucleotide and the labeled oligonucleotide to the target nucleic acid, the polymerase with 5' nuclease activity cleaves the labeled oligonucleotide. This cleavage does not involve the DNA polymerisation. It is preferred that this cleavage occurs before the polymerisations or before the extended strand reaches the helper oligonucleotide. During the hybridisation of the helper and labeled oligonucleotide with the target nucleic acid, the amplification primer may anneal to the same target strand upstream of the helper oligonucleotide. However, the hybridisation condition, for example the temperature, may favour the occurrence of the cleavage of the annealed, labeled oligonucleotide by the polymerase with 5' nuclease activity, not the primer extension. It is also desirable that sequential rounds of the labeled oligonucleotide annealing and cleavage of labeled fragments can occur, that is to say that the cleaved labeled oligonucleotide can depart to allow the binding and cleavage of other copies of the probe in a single hybridisation (or cleavage) step. After the hybridisation step (or cleavage step), the reaction mixture is then put under the extension conditions, for example, the reaction temperature is raised for optimal primer extension. In some circumstances, the optimal conditions for hybridisation, cleavage of the probe and the primer extension may be the same condition, for example the same temperature.

The present invention is compatible, however, with other amplification systems, such as the transcription amplification system, in which one of the amplification primers encodes a promoter that is used to make RNA copies of the target sequence. In a similar fashion, the present invention can be used in a self-sustained sequence replication (3SR) system, in which a variety of enzymes are used to make RNA transcripts that are then used to make DNA copies, all at a single temperature. By incorporating a polymerase with 5' 3' exonuclease activity into a ligase chain reaction (LCR) system, together with appropriate oligonucleotides, one can also employ the present invention to detect LCR products. The present invention can also be used in SDA, LAMP, PCDR, RPA, RCA, ICAN, NASBA, HAD and the like, in a similar fashion.

In either process described herein, a sample is provided which is suspected of containing the particular sequence of interest, the "target nucleic acid". The target nucleic acid contained in the sample may first be reverse transcribed into cDNA, if necessary, and then denatured, using any suitable denaturing method, including physical, chemical, or enzymatic means, which are known to those of skill in the art. A preferred physical means for strand separation involves heating the nucleic acid until it is completely (>99%) denatured. Typical heat denaturation involves temperatures ranging from about 80°C. to about 105°C., for times ranging from a few seconds to minutes. As an alternative to denaturation, the target nucleic acid may exist in a single-stranded form in the sample, such as, single-stranded RNA or DNA viruses.

The denatured nucleic acid strands are then incubated with preselected amplification primers, helper oligonucleotide and labeled oligonucleotide (also referred to herein as "probe") under hybridisation conditions, conditions which enable the binding of the primers, helper oligonucleotide and probes to the single nucleic acid strands. As known in the art, the primers are selected so that their relative positions along a duplex sequence are such that an extension product synthesized from one primer, when the extension product is separated from its template-(complement), serves as a template for the extension of the other primer to yield a replicate chain of defined length. Normally in the hybridisation condition the cleavage of the labeled oligonucleotide occurs. In this case, the hybridisation condition is the same as the cleavage condition. Otherwise, the reaction can be put in a cleavage condition optimal for the cleavage. A separate cleavage condition may comprise a particular temperature optimal for the cleavage of the labeled oligonucleotide.

The primer must be sufficiently long to prime the synthesis of extension products in the presence of the agent for polymerisation. The exact length and composition of the primer will depend on many factors, including temperature of the annealing reaction, source and composition of the primer, proximity of the helper oligonucleotide annealing site to the primer annealing site, and ratio of primer:probe concentration. For example, depending on the complexity of the target sequence, the oligonucleotide primer typically contains about 15-40 nucleotides, although a primer may contain more or less nucleotides. The primers must be sufficiently complementary to anneal to their respective strands selectively and form stable duplexes.

The primers used herein are selected to be "substantially" complementary to the different strands of each specific sequence to be amplified. The primers need not reflect the exact sequence of the template, but must be sufficiently complementary to hybridize selectively to their respective strands. Non-complementary bases or longer sequences can be interspersed into the primer or located at the ends of the primer, provided the primer retains sufficient complementarity with a template strand to form a stable duplex therewith.

An amplification primer can be a target-specific primer, which comprises a 3' priming portion which is complementary to a desired region of target nucleic acid. An amplification primer can also be a universal primer, which has a sequence identical or substantially identical, to a 5' universal portion of target-specific primers. A reaction may contain multiple primers for amplification of multiple target sequences. The 5' universal portions of the multiple target specific primers may have essentially the same sequence composition which is identical, or substantially identical to the universal primer.

For SNP genotyping or detecting variant nucleotides, the amplification primer may be an allele-specific primer, wherein a terminal nucleotide of the primer is selected to be complementary to either the suspected variant nucleotide or the corresponding normal nucleotide such that an extension product of the primer is synthesised when the primer anneals to the diagnostic region containing a particular nucleotide, but no such extension product is synthesised when the primer anneals to the diagnostic region containing no particular nucleotide of the target nucleic acid sequence.

Primer pairs of forward and reverse primers are included in the amplification reaction mixture such that, if a target nucleic acid is present in the sample, the primer pairs are capable of amplifying that target nucleic acid, preferably in an exponential manner.

An amplification primer is designed to anneal to the target nucleic acid sequence upstream of a helper oligonucleotide, which is located upstream or overlaps with the labeled oligonucleotide (the probe). There is no particular distance requirement between the amplification primer and the downstream helper oligonucleotide.

In the practice of the invention, the helper oligonucleotide must first be annealed to the complementary target nucleic acid before the amplification primer anneals to the target nucleotide or before the labeled oligonucleotide anneals to the target nucleic acid. To enhance the likelihood that the helper oligonucleotide will have annealed to a complementary nucleic acid before primer extension polymerisation reaches this duplex region, a variety of techniques may be employed. One can position the helper oligonucleotide so that the 5'-end of the helper oligonucleotide is relatively far from the 3'-end of the amplification primer, thereby giving the helper oligonucleotide more time to anneal before primer extension blocks the helper oligonucleotide binding site. Short primer molecules generally require lower temperatures to form sufficiently stable hybrid complexes with the target nucleic acid. Therefore, the helper oligonucleotide can be designed to be longer than the primer so that the helper oligonucleotide anneals preferentially to the target at higher temperatures relative to primer annealing.

One can also use primers and helper oligonucleotides having differential thermal stability. For example, the nucleotide composition of the helper oligonucleotide can be chosen to have greater G/C content and, consequently, greater thermal stability than the primer. In a similar fashion, one can incorporate modified nucleotides into the helper oligonucleotide Modified nucleotides contain base analogs that form more stable base pairs than the bases that are typically present in naturally occurring nucleic acids.

Modifications of the helper oligonucleotide and/or labeled oligonucleotide (the probe) that may facilitate the oligonucleotide binding prior to primer binding to maximize the efficiency of the present assay include the incorporation of positively charged or neutral phosphodiester linkages in the oligonucleotide to decrease the repulsion of the polyanionic backbones of the oligonucleotide and target (see Letsinger et al., 1988, J. Amer. Chem. Soc: 110:4470); the incorporation of alkylated or halogenated bases, such as 5-bromouridine, in the oligonucleotide to increase base stacking; the incorporation of ribonucleotides into the oligonucleotide to force the oligonucleotide:target duplex into an "A" structure, which has increased base stacking; the substitution of 2,6-diaminopurine (amino adenosine) for some, or all of the adenosines in the oligonucleotide; and the incorporation of nucleotide derivatives such as LNA (locked nucleic acid), PNA (peptide nucleic acid) or the like.

Generally, the 3' terminus of the helper oligonucleotide and/or the probe will be "blocked" to prohibit incorporation of the probe or oligonucleotide, into a primer extension product. However, in some embodiments of the present invention, some probes or oligonucleotides may be working as primers and therefore are not blocked at the 3' terminus. "Blocking" can be achieved by using non-complementary bases or by adding a chemical moiety such as biotin or a phosphate group, to the 3' hydroxyl of the last nucleotide. This may, depending upon the selected moiety, serve a dual purpose by also acting as a label for subsequent detection or capture of the nucleic acid attached to the label. Blocking can also be achieved by removing the 3'-OH or by using a nucleotide that lacks a 3'-OH, such as a dideoxynucleotide.

The thermocycling parameters can also be varied to take advantage of the differential thermal stability of the helper oligonucleotide, the labeled oligonucleotide and primer. For example, following the denaturation step in thermocycling, an intermediate temperature may be introduced which is permissible for helper oligonucleotide and/or labeled oligonucleotide binding but not primer binding, and then the temperature is further reduced to permit primer annealing and extension. One should note, however, that probe cleavage need only occur in later cycles of the PCR process for suitable results. Thus, one could set up the reaction mixture so that even though primers initially bind preferentially to probes, primer concentration is reduced through primer extension so that, in later cycles, probes bind preferentially to primers.

To favour binding of the helper oligonucleotide and/or the labeled oligonucleotide before the primer, a high molar excess of the helper oligonucleotide and the labeled oligonucleotide to primer concentration can also be used. In this embodiment, helper and/or labeled oligonucleotide concentrations are typically in the range of about 2 to 20 times higher than the respective primer concentration. Those of skill recognize that oligonucleotide concentration, length, and base composition are all important factors that affect the Tm of any particular oligonucleotide in a reaction mixture. Each of these factors can be manipulated to create a thermodynamic bias to favour helper oligonucleotide and/or labeled oligonucleotide annealing over primer annealing.

One may also recognize that to facilitate the probe's sequential rounds of annealing and cleavage during the hybridisation/cleavage step at each cycle, the labeled oligonucleotide concentration can be in a range of about 2 to 10 times higher than the respective helper oligonucleotide concentration.

The composition of the labeled oligonucleotide can be designed to favour nuclease activity. Alternatively, the incorporation of modified phosphodiester linkages (e.g., phosphorothioate or methylphosphonates) in the labeled probe during chemical synthesis (Noble et al., 1984, Nuc Acids Res 12:3387-3403; Iyer et al., 1990, J. Am. Chem. Soc. 112:1253-1254) may be used to prevent cleavage at a selected site.

In yet another embodiment, two helper oligonucleotides and two labeled oligonucleotides are used, each complementary to separate regions of separate strands of a double-stranded target region, but not to each other, so that a labeled oligonucleotide anneals adjacent to or overlaps with each helper oligonucleotide. For example, the presence of two labeled oligonucleotides can potentially double the intensity of the signal generated from labels and may further serve to reduce product strand reannealing, as often occurs during PCR amplification. The labeled oligonucleotides are selected so that they bind at positions adjacent to (or overlap with) the positions at which helper oligonucleotides bind.

One can also use multiple helper oligonucleotides and multiple labeled oligonucleotides in the present invention to achieve other benefits. For instance, one could test for any number of pathogens in a sample simply by putting as many helper oligonucleotides and labeled oligonucleotides as desired into the reaction mixture; the probes could each comprise a different label to facilitate detection.

One can also achieve allele-specific or species-specific discrimination using multiple labeled oligonucleotides in the present invention. For instance, by using probes that have different Tₘs and conducting the annealing/cleavage reaction at a temperature specific for only one probe/allele duplex.

Also disclosed is an amplification process for detecting SNP or a mutation in a target nucleic acid sequence, wherein the nucleotide in the labeled oligonucleotide, complementary to the SNP or the mutated nucleotide is susceptible to be cleaved by the 5' nuclease activity of a polymerase.

It is preferred that multiple nucleotides or phosphodiester linkages 3'of the nucleotide complementary to the SNP, or the mutated nucleotide, are resistant to be cleaved by the 5' nuclease activity of a polymerase. Alternatively, at least one nucleotide immediately downstream 3' of the nucleotide complementary to the SNP or the mutated nucleotide, is resistant to being cleaved by the 5' nuclease activity of a polymerase. To achieve the resistance of nuclease cleavage, the nucleotide or linkage 3'of the nucleotide complementary to the SNP or the mutated nucleotide is modified; such modified linkage may be phosphorothioate linkage.

In one embodiment, the nucleotide in the labeled oligonucleotide complementary to the SNP or the mutated nucleotide is the 5' terminal nucleotide, which is preferably attached with a label. In another embodiment, the nucleotide complementary to the SNP or the mutated nucleotide is internally located in the labeled oligonucleotide, which may not be attached with a label. In this case, the labeled oligonucleotide may comprise a 5' non-complementary tail which is attached with a label.

In an amplification reaction, the labeled oligonucleotide with a sequence complementary to a diagnostic region containing a SNP or mutated nucleotide, anneals to the target nucleic acid sequence; a helper oligonucleotide anneals to the target nucleic acid sequence adjacent to the labeled oligonucleotide, and optionally, the amplification primer may anneal to the same target strand upstream of the helper oligonucleotide. If the nucleotide in the labeled oligonucleotide is complementary to the SNP or the mutated nucleotide in the target nucleic acid sequence, the 5' nuclease of a polymerase cleaves the annealed, labeled oligonucleotide. The cleavage site is preferably designed to occur at the 3' of the nucleotide which is complementary to the SNP or the mutated nucleotide. It is also designed so that if there is a mismatch at the site of SNP or the mutated nucleotide, there will be no cleavage. So the phosphodiester linkage 3'of the nucleotide which is complementary to the SNP or the mutated nucleotide, is designed to be susceptible to the cleavage. It is preferred that to prevent a non-specific cleavage, the multiple nucleotides or linkages downstream of the nucleotide complementary to the SNP or the mutated nucleotide are resistant to being cleaved by the 5' nuclease activity of a polymerase (Fig. 2). Alternatively, at least one nucleotide immediately downstream of the nucleotide complementary to the SNP or the mutated nucleotide should be resistant to being cleaved by the 5' nuclease activity of a polymerase. This modification is necessary, because during the extension of the amplification primer, if the probe anneals to the target which does not contain the appropriate SNP, the 5' nuclease activity of the polymerase may trim the probe, therefore resulting in a false signal. It is also possible that by optimization of the reaction condition, for example, by raising the reaction temperature during the extension step, during which the probe is dissociated from the template, the 5' nuclease activity of the polymerase cannot trim the probe. In this case, the modification of the probe may be unnecessary.

One can achieve allele specific discrimination by using probes with different labels each label specific for each allele. The nucleotide in the labeled oligonucleotide complementary to the SNP or the mutated nucleotide is susceptible to being cleaved by the 5' nuclease of a polymerase.

Template-dependent extension of the oligonucleotide primer(s) is catalyzed by a polymerizing agent in the presence of adequate amounts of the four deoxyribonucleoside triphosphates (dATP, dGTP, dCIP, and dTTP) or analogs as discussed above, in a reaction medium comprised of the appropriate salts, metal cations, and pH buffering system. Suitable polymerizing agents are enzymes known to catalyze primer- and template-dependent DNA synthesis and possess the 5' nuclease activity. Known DNA polymerases include, for example, E, coli DNA polymerase I, Thermus thermophilus (Tth) DNA polymerase, Bacillus stearothermophilus DNA-polymerase, Thermococcus litoralis DNA polymerase, and Thermus aquaticus (Taq) DNA polymerase. The reaction conditions for catalyzing DNA synthesis with these DNA polymerases are well known in the art. To be useful in the present invention, the polymerizing and/or cleavage agent must efficiently cleave the oligonucleotide and release labeled fragments so that the signal is directly or indirectly generated.

In a preferred method, the PCR process is carried out as an automated process that utilizes a thermostable enzyme. In this process, the reaction mixture is cycled through a denaturing step, a probe and primer annealing step, and a synthesis step. Temperature stable polymerases are preferred in this automated process. It is also preferred that the annealing step and the synthesis step are repeated at least once within the single round of thermal cycling.

Reagents employed in the methods of the invention can be packaged into diagnostic kits. Diagnostic kits include the labeled oligonucleotides, the helper oligonucleotides and the primers, in separate containers or in a single master mixture container. If the oligonucleotide is unlabeled, the specific labeling reagents may also be included in the kit. The kit may also contain other suitably packaged reagents and materials needed for amplification, for example, buffers, dNTPs, and/or polymerizing means and the cleavage agent; and for detection analysis, for example, enzymes and solid phase extractants, as well as instructions for conducting the assay.

In another aspect, the invention provides a method for monitoring nucleic acid amplification comprising (e.g. as shown in Fig. 4B):
(a) providing to an amplification assay containing said sample:
   (i) a pair of amplification primers,
      wherein the first amplification primer contains a sequence complementary to a region of one strand of the target nucleic acid and is capable of priming the synthesis of the complementary strand,
      and wherein the second primer contains a sequence complementary to a region of the second strand of the target nucleic acid and is capable of priming the synthesis of a strand which is complementary thereto,
      such that, upon amplification in the presence of the target nucleic acid, the stretch of target nucleic acid between the amplification primers is amplified;
   (ii) a set of multiple labeled oligonucleotides, wherein each of the labeled oligonucleotides anneals to the same strand of the target nucleic acid, wherein all the labeled oligonucleotides anneal within the target nucleic acid region comprised between the binding regions of the amplification primers,
   wherein
   if the target nucleic acid is present in the sample, the labeled oligonucleotides anneal to the target nucleic acid such that the 3' end of one of the labeled oligonucleotide is adjacent to or overlaps the 5' end of a downstream labeled oligonucleotide, one of the amplification primers is adjacent to or overlaps the 5' end of a downstream labeled oligonucleotide;
(b) carrying out an amplification reaction using a nucleic acid polymerase having 5' nuclease activity under conditions which would, if the target nucleic acid is present in the sample, be permissive for the steps of:
   (i) hybridising the amplification primers, labeled oligonucleotides to the target nucleic acid,
      wherein the annealed upstream labeled oligonucleotide acts as helper oligonucleotide, facilities the hybridisation of the downstream labeled oligonucleotide and/or enhances the cleavage of the annealed, labeled oligonucleotide by the 5' nuclease activity of the polymerase, in a polymerization independent manner, to release labeled fragments which are detectable; and (ii) extending the amplification primers, wherein the nucleic acid polymerase synthesizes primer extension products,
      wherein the hybridisation condition and/or extension condition permits the 5' nuclease activity of the polymerase to cleave the annealed, labeled oligonucleotide and release labeled fragments; and (c) detecting and/or measuring the release of labeled fragments to determine the presence or absence or amount of the target nucleic acid sequence in the sample.

The process may be performed under conditions sufficient to promote nucleic acid polymerization, wherein the release of labeled fragments occurs during extension of the oligonucleotide primer.

The multiple labeled oligonucleotides anneal to the target nucleic acid sequence adjacent to each other or with a small overlapping region. The gap or the overlapping space between the labeled oligonucleotides or between primer and one labeled oligonucleotide permit the cleavage of the labeled oligonucleotide in the absence of nucleic acid polymerization. Alternatively, the cleavage of the labeled oligonucleotide occurs during extension of the amplification primer. The labeled oligonucleotides in the set may comprise the same labels or different labels. A reaction may comprise a pair of competing labeled oligonucleotides, wherein one labeled oligonucleotide of the pair is complementary to the wild type target sequence, the second labeled oligonucleotide in the pair is complementary to target sequence with variant nucleotides.

In another aspect, disclosed herein is a process for monitoring nucleic acid amplification comprising:
Performing nucleic acid amplification on a target nucleic acid using
   a nucleic acid polymerase having 5' nuclease activity,
   a helper oligonucleotide containing a sequence complementary to a region of the target nucleic acid,
   a labeled oligonucleotide containing a sequence complementary to a second region of the same target nucleic acid sequence strand, but not including or partially including the nucleic acid defined by the helper oligonucleotide, to create a mixture of duplexes during hybridisation conditions, if the target nucleic acid sequence is present in the sample, wherein the duplexes comprise the target nucleic acid annealed to the helper oligonucleotide and to the labeled oligonucleotide such that the 3' end of the helper oligonucleotide is adjacent to or overlaps the 5' end of the labeled oligonucleotide, wherein said helper oligonucleotide and said labeled oligonucleotide anneals within the target nucleic acid sequence bound by the amplification primers as defined below,
   a set of amplification primers, wherein a first amplification primer contains a sequence complementary to a region in one strand of the target nucleic acid sequence and primes the synthesis of a complementary strand, and a second primer contains a sequence complementary to a region in a second strand of the target nucleic acid sequence and primes the synthesis of a complementary strand; and wherein each amplification primer is selected to anneal to its complementary template upstream of any helper oligonucleotide annealed to the same nucleic acid strand,
   said labeled oligonucleotide having a fluorescent reporter molecule and a quencher molecule capable of quenching the fluorescence of said reporter molecule, alternatively, the quencher is located on a separate oligonucleotide having a sequence complementary to the labeled oligonucleotide,
   said nucleic acid polymerase cleaving the annealed, labeled oligonucleotide during amplification to separate said reporter molecule from said quencher molecule; and monitoring the fluorescence of said reporter molecule.

In a further aspect, disclosed herein is a process for the detection or measurement of a target nucleic acid sequence in a sample, said process comprising:
(a) contacting a sample comprising single-stranded nucleic acids with an first oligonucleotide primer containing a sequence complementary to a first region of the target nucleic acid and a labeled oligonucleotide containing a sequence complementary to a second region of the same target nucleic acid sequence strand, overlapping the first region defined by the first oligonucleotide primer, to create a mixture of duplexes during hybridization conditions, if the target nucleic acid sequence is present in the sample, wherein the duplexes comprise the target nucleic acid annealed to the first oligonucleotide primer and to the labeled oligonucleotide such that the 3' end of the first oligonucleotide primer overlaps the 5' end of the labeled oligonucleotide;
(b) maintaining the mixture of step (a) with a template-dependent nucleic acid polymerase having a 5' to 3' nuclease activity under conditions sufficient to permit the 5' to 3' nuclease activity of the polymerase to cleave the annealed, labeled oligonucleotide and release labeled fragments; and
(c) detecting and/or measuring the release of labeled fragments and thereby detecting and/or measuring the target nucleic acid in the sample.

In the above process, the 3' end of the first oligonucleotide primer in the annealed duplex of step (a) overlaps the 5' end of an annealed, labeled oligonucleotide, having overlapping length effective to permit the release of labeled fragments in the absence of nucleic acid polymerization. The process may be performed under conditions sufficient to promote nucleic acid polymerization, wherein the release of labeled fragments occurs during extension of the first oligonucleotide primer.

In another aspect, also disclosed herein is a polymerase chain reaction (PCR) amplification process for detecting a target nucleic acid sequence in a sample, said process comprising:
(a) providing to a PCR assay containing said sample, a least one labeled oligonucleotide containing a sequence complementary to a region of the target nucleic acid, wherein said labeled oligonucleotide anneals within the target nucleic acid sequence bounded by the oligonucleotide primers of step (b);
(b) providing a set of oligonucleotide primers, wherein a first primer contains a sequence complementary to a region in one strand of the target nucleic acid sequence and primes the synthesis of a complementary DNA strand, and a second primer contains a sequence complementary to a region in a second strand of the target nucleic acid sequence and primes the synthesis of a complementary DNA strand; and wherein at least one of the oligonucleotide primers is selected to anneal to its complementary region overlapping one of the labeled oligonucleotides annealed to the same nucleic acid strand and wherein the 3' end of an annealed oligonucleotide primer overlaps the 5' end of said labeled oligonucleotide annealed to the same nucleic acid strand;
(c) amplifying the target nucleic acid sequence, if the target sequence is present in the sample, employing a nucleic acid polymerase having 5' to 3' nuclease activity as a template-dependent polymerizing agent under conditions which are permissive for PCR cycling steps of (i) annealing of primers and labeled oligonucleotide to a template nucleic acid sequence contained within the target sequence, wherein the 5' to 3' nuclease activity of the nucleic acid polymerase cleave the annealed, labeled oligonucleotide in the polymerization independent manner, and (ii) extending the primer wherein said nucleic acid polymerase synthesizes a primer extension product while the 5' to 3' nuclease activity of the nucleic acid polymerase simultaneously releases labeled fragments from the annealed duplexes comprising labeled oligonucleotide and its complementary template nucleic acid sequences, thereby creating detectable labeled fragments; and
(d) detecting and/or measuring the release of labeled fragments to determine the presence or absence of the target sequence in the sample.

In the above process, the overlapping length of the 3' end of the first oligonucleotide primer and the 5' end of the labeled oligonucleotide is from 1 to 15 nucleotides long. Preferably, the overlapping length of the 3' end of the first oligonucleotide primer and the 5' end of the labeled oligonucleotide is 1 nucleotide long.

In a further aspect, also disclosed herein is a method using a novel thermal profile for improved PCR amplification, said novel thermal profile comprising:
(a) denaturing double-stranded nucleic acid fragment at denaturing conditions;
(b) hybridising probes/primers to the target nucleic acid at hybridising conditions;
(c) extending hybridised primers or dissociating hybridised probe/primer from target nucleic acid at extending conditions,
(d) repeating (b) and (c) at least once; and
(e) repeating (a) to (d) at least 5 times,
wherein said denaturing conditions comprise high temperatures, which preferably are between 93°C - 100°C, wherein said hybridising conditions comprise appropriate temperatures, which preferably are between 35°C - 65°C, at the hybridising conditions the labeled oligonucleotide (the probe) and helper oligonucleotide are hybridised to the target nucleic acid and the annealed labeled oligonucleotides are preferably cleaved by a nuclease activity of the polymerase, in a polymerisation independent manner, to release labeled fragments which are detectable, wherein in the step(c) either the hybridised probe/primer dissociated from template or hybridised primers extended or both.

It is preferred that, as the steps (b)-(c) are repeated at least once within single round of thermal cycling, in the first repeat of (b) and (c), probes or primers hybridise to the target nucleic acid at hybridising conditions, wherein said hybridising conditions comprise a temperature which is not optimal for hybridisation of amplification primers, but allows efficient hybridisation of the labeled oligonucleotide (the probe) and helper oligonucleotide, and promote cleavage of the labeled oligonucleotide (the probe). In this situation in the step (c) the primer extending does not occur at said extending conditions, but annealed probes may be dissociated.

In the second repeat of (b) and (c) of the step (d), the hybridising conditions may comprise a temperature which is optimal for hybridisation of amplification primers and other probes/primers. In this situation in the step (c) the primer extending does occur at said extending conditions, and cleavage of the probe also occurs.

Also disclosed herein is a kit for detecting a nucleic acid sequence in a sample comprising:
(a) a set of oligonucleotide primers, wherein a first primer contains a sequence complementary to a region in one strand of the nucleic acid sequence and primes the synthesis of a first extension product, and a second primer contains a sequence complementary to a region in said first extension product and primes the synthesis of a nucleic acid strand complementary to said first extension product, and wherein each oligonucleotide primer is selected to anneal to its complementary template upstream of any labeled oligonucleotide annealed to the same nucleic acid strand,
(b) at least one helper oligonucleotide containing a sequence complementary to a region of the target nucleic acid and at least one labeled oligonucleotide containing a sequence complementary to a second region of the same target nucleic acid sequence strand, but not including, or partially including the nucleic acid defined by the helper oligonucleotide, wherein said helper oligonucleotide and said labeled oligonucleotide are capable of hybridising to the target sequence to create duplexes during hybridisation conditions, wherein the duplexes comprise the target nucleic acid annealed to both the helper oligonucleotide and the labeled oligonucleotide such that the 3' end of the helper oligonucleotide is adjacent to or overlaps with the 5' end of the labeled oligonucleotide, wherein said helper oligonucleotide and said labeled oligonucleotide anneal within the target nucleic acid sequence bound by the amplification primers of part (a), wherein said labeled oligonucleotide is blocked at the 3' terminus to prohibit incorporation of said labeled oligonucleotide into a primer extension product,
wherein said blocking is achieved by adding a chemical moiety to the 3' hydroxyl of the last nucleotide,
wherein said labeled oligonucleotide is provided in a soluble form and it generates a signal concurrently with the amplification of said nucleic acid sequence using said set of oligonucleotide primers,
wherein said labeled oligonucleotide is coupled to a solid support,
wherein said labeled oligonucleotide and said helper oligonucleotide anneal within the nucleic acid sequence concurrently with each oligonucleotide primer priming the synthesis of the first extension product or the nucleic acid strand complementary to the first extension product.
wherein said labeled oligonucleotide is blocked at the 3' terminus by adding a chemical moiety to the 3' hydroxyl of the last nucleotide of said labeled oligonucleotide, which chemical moiety is a phosphate group.
wherein said labeled oligonucleotide is blocked at the 3' terminus by removing the 3'-hydroxyl from said labeled oligonucleotide.
wherein said labeled oligonucleotide contains at least two labels.
wherein said labeled oligonucleotide contains a label at its 5' end or 3' end.
wherein said labeled oligonucleotide contains a first label and a second label, and wherein the first label and the second label interact with each other.
wherein said labeled oligonucleotide contains a first label and a second label and wherein the first label and the second label are separated from each other by a nuclease susceptible cleavage site.
wherein the first label is a fluorophore and the second label is a quencher.

The kit may comprise a nucleic acid polymerase having a 5' to 3' nuclease activity.

### BRIEF DESCRIPTION OF THE FIGURES

FIG. 1 graphically presents the annealing of the amplification primer, helper oligonucleotide and labeled oligonucleotide to the target nucleic acid sequence. (A) the helper oligonucleotide anneals upstream of the labeled oligonucleotide with the gap of 1 nucleotide which is for illustration purpose (the gap with 0 to 10 nucleotides is preferred); (B) the helper oligonucleotide anneals to the target sequence, overlapping with the labeled oligonucleotide with the overlapping space of 3 nucleotides for illustration purposes (the overlapping space with 1 to 10 nucleotides is preferred); (C) the helper oligonucleotide anneals to the target sequence, overlapping with the labeled oligonucleotide with the overlapping space of 2 nucleotides and with a non-complementary 3' terminus nucleotide of the helper oligonucleotide; (D) the helper oligonucleotide anneals to the target sequence, overlapping with the labeled oligonucleotide with the overlapping space of 2 nucleotides and with a non-complementary 3' terminus nucleotide of the helper oligonucleotide; the labeled oligonucleotide contains a non-complementary 5' tail.
FIG. 2 graphically presents an embodiment for detecting a SNP or mutated nucleotide. The helper oligonucleotide anneals to the target nucleic acid sequence adjacent to the labeled oligonucleotide without a gap. The 3' terminus nucleotide of the helper oligonucleotide is not complementary to the SNP or the mutated nucleotide on the target sequence. The 5' terminus nucleotide of the labeled oligonucleotide is complementary to the SNP or the mutated nucleotide, and is capable of being cleaved by a cleavage agent, the 5' nuclease of polymerase. The two linkages linking the nucleotides 3' to the 5' terminus nucleotide are modified to prevent cleavage.

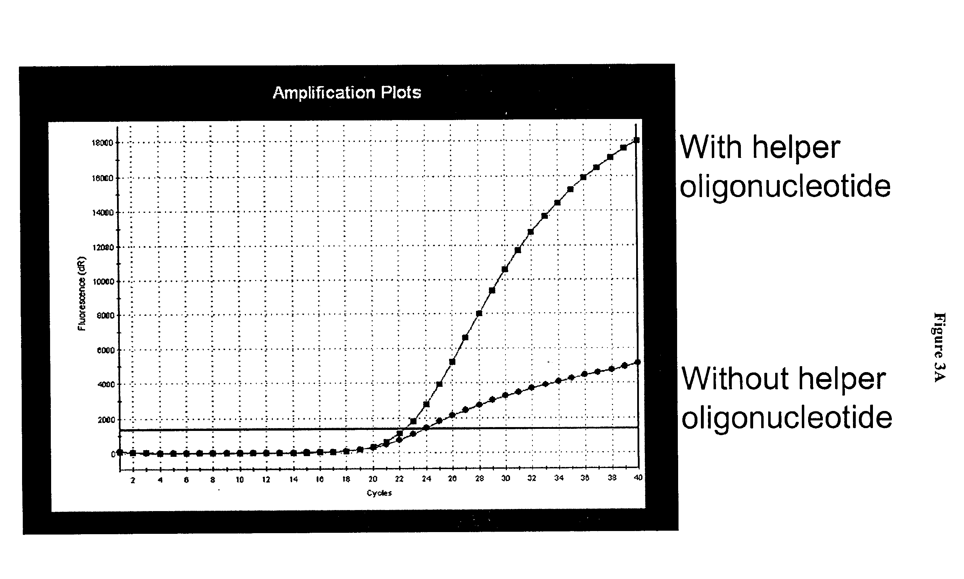

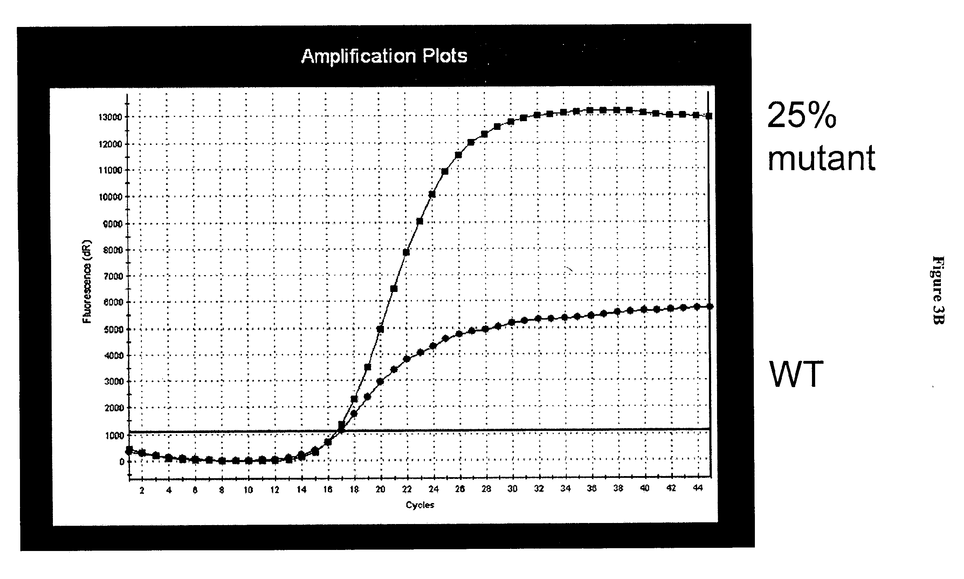

FIG. 3 show amplification plots of experiments carried out in Sample 1 and 2.
FIG. 4 (A) graphically presents an embodiment for using amplification primer annealed to the target nucleic acid sequence overlapping with a labeled oligonucleotide. (B) shows multiple labeled oligonucleotides anneal to the same strand of a target nucleic acid sequence adjacent to or overlap each other.

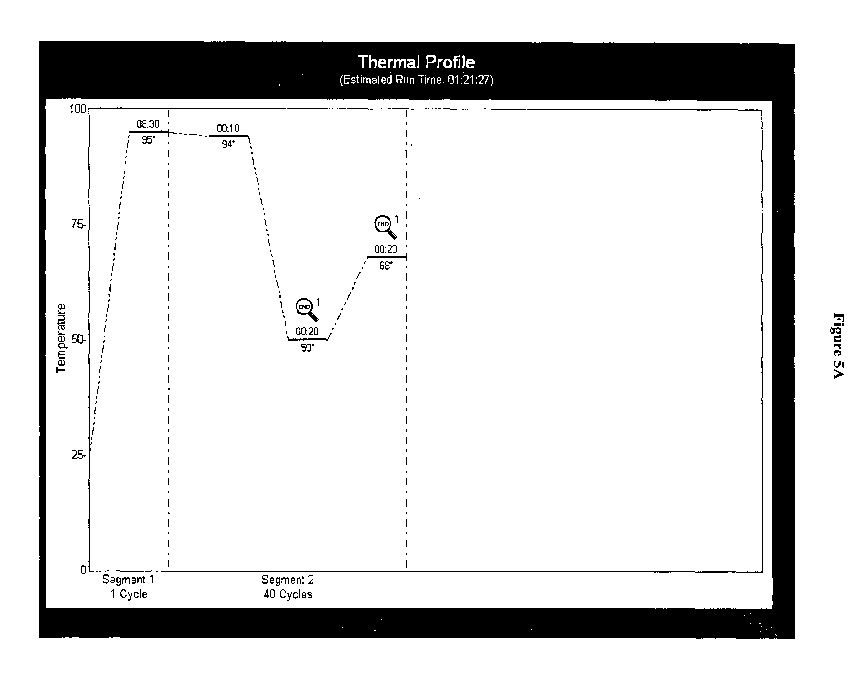

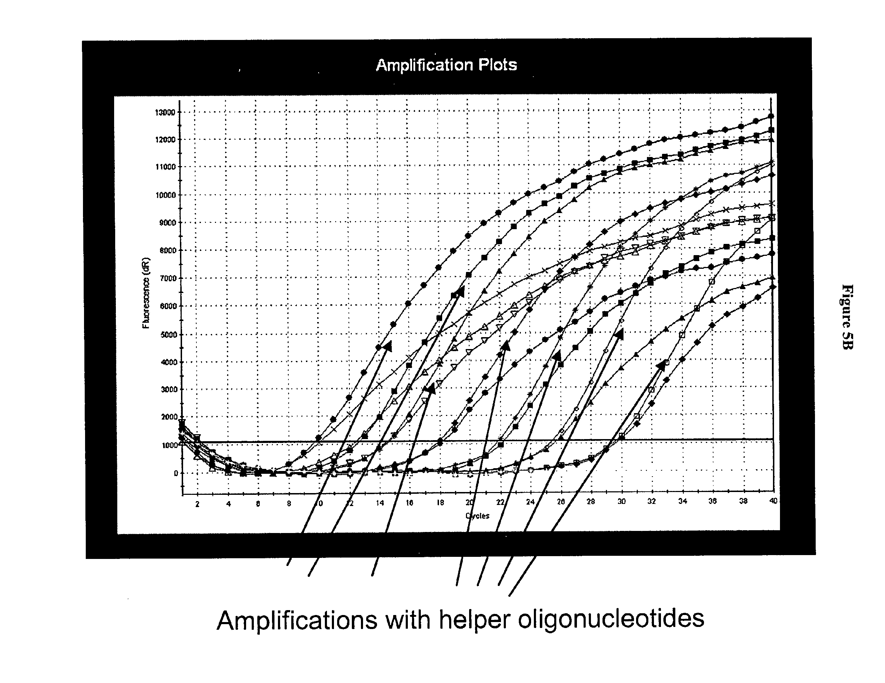

FIG. 5 show amplification plots of experiments carried out in Sample 3.

### EXAMPLES

The present invention is exemplified by the following Examples, in which parts and percentages are by weight and degrees are Celsius, unless otherwise stated. It should be understood that these Examples, while indicating preferred embodiments of the invention, are given by way of illustration only.

### Example 1

PCR amplifications were performed with helper oligonucleotide and without helper oligonucleotide. The sequences of the primers and probes were as follows:
SEQ ID NO 1 CCTTTACTTACTACACCTCAGA (SEQ ID NO 1)
SEQ ID NO 2 GCCTCAATTCTTACCATCCACAA (SEQ ID NO 2)
SEQ ID NO 3 cTCCCATCAGTTTGAACAGTTGTCTGGgag (SEQ ID NO 3)
SEQ ID NO 4 TGAAATCTCGATGGAGTGGG (SEQ ID NO 4)

SEQ ID NO 1 is forward amplification primer; SEQ ID NO 2 is reverse amplification primer; SEQ ID NO 3 is labeled oligonucleotide (probe) with Joe attached at 5' end, BHQ-1 attached at the 3' end. SEQ ID NO 4 is helper oligonucleotide with 3' end blocked with phosphate.

Oligonucleotides were diluted to a final concentration of 10 µM. Amplification was performed using the following ingredients and conditions: 1XPCR Buffer (Roche FastStart Taq DNA Polymerase), 1XGC solution, 0.2 mM dNTPs, 0.3µM of each primer, 0.8 µM of helper oligonucleotide, if added, FastStart Taq DNA polymerase (0.1 U/µl), plasmid DNA 0.5 µl (10⁵ molecules) and water to final volume of 25 µl. Reactions are carried out at 94 °C for 4 min; 10 cycles of 20 sec at 95°C, 20 sec at 59°C, 20 sec at 72°C, 40 cycles of 20 sec at 95°C, 20 sec at 57°C, 20 sec at 72°C, 20 sec at 53°C, 30 sec at 68°C (data being collected at each temperature) on a Stratagene MX3005 real-time PCR machine. The plasmid template contains a fragment of human BRAF sequence. The helper oligonucleotide hybridizes next to the probe. During the annealing step, the Taq polymerase with the 5' nuclease may cleave the probe in the polymerization independent manner.

Figure 3A shows the result of PCR amplification with the helper oligonucleotide and without the helper oligonucleotide.

### Example 2

PCR amplifications were performed to amplify a genomic region of Kras gene with a point mutation of codon 12 GGT-GTT. The sequences of the primers and probes were as follows:
SEQ ID NO: 5 GTCACATTTTCATTATTTTTATTATAAGGCCTGC (SEQ ID NO 5)
SEQ ID NO: 6 CTCTATTGTTGGATCATATTCGTCCACA (SEQ ID NO 6)
SEQ ID NO: 7 GTCAAGGCACTCTTGCCTACGCCAA (SEQ ID NO 7)
SEQ ID NO: 8 CCAGCTCCAACTACCACAAGT (SEQ ID NO 8)

SEQ ID NO 5 is forward amplification primer; SEQ ID NO 6 is reverse amplification primer; SEQ ID NO 7 is helper oligonucleotide; SEQ ID NO 8 is oligonucleotide (probe) with HEX attached at 5' end, BHQ-1 attached at the 3' end.

Oligonucleotides were diluted to a final concentration of 10 µM. Amplification was performed using the following ingredients and conditions: 1XPCR Buffer (Roche FastStart Taq DNA Polymerase), 1XGC solution, 0.2 mM dNTPs, 0.3µM of each primer, 0.8 µM of helper oligonucleotide, if added, FastStart Taq DNA polymerase (0.1 U/µl), plasmid DNA 0.5 µl (105 molecules) and water to final volume of 25 µl. Reactions are carried out at 94 °C for 4 min; 10 cycles of 15 sec at 95°C, 20 sec at 60°C, 20 sec at 72°C, 45 cycles of 15 sec at 95°C, 20 sec at 57°C, 20 sec at 72°C, 20 sec at 54°C, 30 sec at 68°C (data being collected at each temperature) on a Stratagene MX3005 real-time PCR machine. The plasmid template contains a fragment of human Kras sequence. The helper oligonucleotide hybridizes next to the probe. During the annealing step, the Taq polymerase with the 5' nuclease may cleave the probe in the polymerization independent manner.

Figure 3B shows the result of PCR amplification with the helper oligonucleotide and varied amount of mutant and wild-type plasmid DNA.

### Example 3

PCR amplifications were performed to amplify a genomic region of Jak2 gene. The sequences of the primers and probes were as follows:
SEQ ID NO: 9 GTGGAGACGAGAGTAAGTAAAACTACA (SEQ ID NO: 9)
SEQ ID NO: 10 CTCCTGTTAAATTATAGTTTACACTGACA (SEQ ID NO: 10)
SEQ ID NO: 11 GATCCTGAAACTGAATTTTCTATATAAACAAAC (SEQ ID NO: 11)
SEQ ID NO: 12 AACAGATGCTCTGAGAAAGGCATTAGA (SEQ ID NO: 12)
SEQ ID NO: 13 CTCAGAGCATCTGTT (SEQ ID NO: 13)

SEQ ID NO 9 is forward amplification primer; SEQ ID NO 10 is reverse amplification primer; SEQ ID NO 11 is helper oligonucleotide; SEQ ID NO 12 is the first oligonucleotide of probe with Fam attached at 5' end, phosphate attached at the 3' end which makes the oligonucleotide unextendable; SEQ ID NO 13 is the second oligonucleotide of probe with DABCYL attached at the 3' end. The first and second oligonucleotides of probe can bind together. In the absence of target, the formation of the first and the second oligonucleotide hybrid brings the quencher and the fluorophore into close proximity, efficiently quenching the fluorescent signal. In the presence of the target, the first oligonucleotide preferentially hybridises to the target sequence and incorporates into the amplicon. As a result, the quencher is separated from the fluorophore resulting in an increase in fluorescence emission.

Oligonucleotides were diluted to a final concentration of 10 µM. Primer-probe master mix was set up as follows: the primers and probes were mixed to a final concentration 0.4 µM of probes and 0.6 µM of primers, which creates a 2X primer-probe master mix. The first mix contains helper oligonucleotide; the second mix contains no helper oligonucleotide.

The reaction mix was created by combining equal amount of 2Xprimer-probe master mix and 2X TaqMan® Gene Expression Master Mix (Applied Biosystem, cat. No 4369514).

Fast reactions were carried out using a thermal profile as shown in Figure 5B. at 94 °C for 8:30 min; 40 cycles of 10 sec at 94°C, 20 sec at 50°C, 20 sec at 68°C; data being collected at 50°C on a Stratagene MX3005 real-time PCR machine. Slow reactions were carried out using a thermal profile as at 94 °C for 8:30 min; 40 cycles of 10 sec at 94°C, 45sec at 50°C, 45 sec at 68°C; data being collected at 50°C on a Stratagene MX3005.

Template DNAs containing target 1, target 2, and mixed target 1 and 2 were serially diluted as follows: 1, 0.1, 0.01, 0.001, 0.0001, 0.00001, 0.000001. no DNA

Figure 5B shows the results of the fast PCR amplification with the helper oligonucleotide and without the helper oligonucleotide. The amplification with helper oligonucleotide gave enhanced fluorescence signals. Comparison of fast and slow reaction results showed that the helper oligonucleotide enhanced both fast and slow reactions, but was more beneficial for fast reactions.

### SEQUENCE LISTING FREE TEXT

SEQ ID NO: 1 <223> Forward amplification primer
SEQ ID NO: 2 <223> Reverse amplification primer
SEQ ID NO: 3 <223> Labelled oligonucleotide probe
SEQ ID NO: 4 <223> Helper oligonucleotide
SEQ ID NO: 5 <223> Forward amplification primer
SEQ ID NO: 6 <223> Reverse amplification primer
SEQ ID NO: 7 <223> Helper oligonucleotide
SEQ ID NO: 8 <223> Oligonucleotide probe
SEQ ID NO: 9 <223> Forward amplification primer
SEQ ID NO: 10 <223> Reverse amplification primer
SEQ ID NO: 11 <223> Helper oligonucleotide
SEQ ID NO: 12 <223> Oligonucleotide probe
SEQ ID NO: 13 <223> Oligonucleotide probe

## Claims

1. A method for the detection or measurement of a target nucleic acid sequence in a sample, said process comprising:
(a) providing to an amplification assay containing said sample:
(i) a pair of amplification primers,
wherein the first amplification primer contains a sequence complementary to a region of one strand of the target nucleic acid and is capable of priming the synthesis of the complementary strand,
and wherein the second primer contains a sequence complementary to a region of the second strand of the target nucleic acid and is capable of priming the synthesis of a strand which is complementary thereto,
such that, upon amplification in the presence of the target nucleic acid, the stretch of target nucleic acid between the amplification primers is amplified;
(ii) a helper oligonucleotide containing a sequence complementary to a first region of the target nucleic acid;
(iii) a labelled oligonucleotide containing a sequence complementary to a second region of the target nucleic acid;
wherein the helper oligonucleotide and the labelled oligonucleotide both anneal to the same strand of the target nucleic acid,
wherein the second region is adjacent to 5' end of the first region, or partially including the first region, and
wherein the helper oligonucleotide and the labelled oligonucleotide both anneal within the target nucleic acid region comprised between the binding regions of the amplification primers,
wherein
if the target nucleic acid is present in the sample, the helper oligonucleotide and the labelled oligonucleotide anneal to the target nucleic acid such that the 3' end of the helper oligonucleotide is adjacent to or overlaps the 5' end of the labelled oligonucleotide,
(b) carrying out an amplification reaction using a nucleic acid polymerase having 5' nuclease activity under conditions which would, if the target nucleic acid is present in the sample, be permissive for the steps of:
(i) hybridising the amplification primers, helper oligonucleotide and labelled oligonucleotide to the target nucleic acid,
wherein the 5' nuclease activity of the polymerase cleaves the annealed, labelled oligonucleotide, in a polymerisation independent manner, to release labelled fragments which are detectable; and
(ii) extending the amplification primers, wherein the nucleic acid polymerase synthesizes primer extension products; and
(c) detecting and/or measuring the release of labelled fragments to determine the presence or absence or amount of the target nucleic acid sequence in the sample.

2. The method of claim 1, wherein the annealed helper oligonucleotide facilities the hybridisation of the labeled oligonucleotide and/or enhances the cleavage of the annealed, labeled oligonucleotide, wherein the hybridising condition and/or extending condition permits the 5' nuclease activity of the polymerase to cleave the annealed, labeled oligonucleotide and release labeled fragments.

3. The method of claim 1 or claim 2, wherein the cleavage of the labeled oligonucleotide occurs during extension of the amplification.

4. The method of any one of claims 1 to 3, wherein either:
(A) the hybridising and extending occur in the same conditions; or
(B) the hybridising condition and the extending condition are repeated at least once in a single round of thermal cycling, if the hybridising and the extending conditions are not the same conditions.

5. The method of any one of claims 1 to 4, wherein either:
(A) the 3' end of the helper oligonucleotide in the annealed duplex is adjacent to the 5' end of an annealed, labeled oligonucleotide, having spacing effective to permit the cleavage of the labeled oligonucleotide in the absence of nucleic acid polymerisation, wherein said spacing between the 3' end of the helper oligonucleotide in the annealed duplex and the 5' end of an annealed, labeled oligonucleotide is from 0 to 10 nucleotides; or
(B) the 3' end of the helper oligonucleotide in the annealed duplex overlaps the 5' end of an annealed, labeled oligonucleotide, having overlapping region effective to permit the cleavage of the labeled oligonucleotide in the absence of nucleic acid polymerisation, wherein said overlapping region between the 3' end of the helper oligonucleotide in the annealed duplex and the 5' end of an annealed, labeled oligonucleotide has 1 to 15 nucleotides.

6. The method of any one of the preceding claims, wherein the 3' end of the helper oligonucleotide in the annealed duplex has a single nucleotide at 3' terminus not complementary to the target nucleic acid sequence.

7. The method of any one of the preceding claims, wherein the labeled oligonucleotide comprises at least one label, wherein the labeled oligonucleotide comprises first and second labels wherein the first label is separated from the second label by a nuclease susceptible cleavage site.

8. The method of any one of the preceding claims, wherein the helper oligonucleotide is not extended during amplification.

9. The method of any one of the preceding claims, wherein said labeled oligonucleotide is a single-stranded double-dye labeled TaqMan probe, or is a molecular beacon probe, or is one of strands of a partially double stranded probe, wherein said partially double-stranded probe comprises first oligonucleotide which is capable of hybridising to second oligonucleotide, wherein each of first and second oligonucleotides comprises a member of interactive label pair.

10. A method for monitoring nucleic acid amplification comprising:
(a) providing to an amplification assay containing said sample:
(i) a pair of amplification primers,
wherein the first amplification primer contains a sequence complementary to a region of one strand of the target nucleic acid and is capable of priming the synthesis of the complementary strand,
and wherein the second primer contains a sequence complementary to a region of the second strand of the target nucleic acid and is capable of priming the synthesis of a strand which is complementary thereto,
such that, upon amplification in the presence of the target nucleic acid, the stretch of target nucleic acid between the amplification primers is amplified;
(ii) a set of multiple labeled oligonucleotides, wherein each of the labeled oligonucleotides anneals to the same strand of the target nucleic acid,
wherein all the labeled oligonucleotides anneal within the target nucleic acid region comprised between the binding regions of the amplification primers,
wherein
if the target nucleic acid is present in the sample, the labeled oligonucleotides anneal to the target nucleic acid such that the 3' end of one of the labeled oligonucleotide is adjacent to or overlaps the 5' end of a downstream labeled oligonucleotide, one of the amplification primers is adjacent to or overlaps the 5' end of a downstream labeled oligonucleotide;
(b) carrying out an amplification reaction using a nucleic acid polymerase having 5' nuclease activity under conditions which would, if the target nucleic acid is present in the sample, be permissive for the steps of:
(i) hybridising the amplification primers, labeled oligonucleotides to the target nucleic acid,
wherein the annealed upstream labeled oligonucleotide acts as helper oligonucleotide, facilities the hybridisation of the downstream labeled oligonucleotide and/or enhances the cleavage of the annealed, labeled oligonucleotide by the 5' nuclease activity of the polymerase, in a polymerisation independent manner, to release labeled fragments which are detectable; and
(ii) extending the amplification primers, wherein the nucleic acid polymerase synthesizes primer extension products,
wherein the hybridising condition and/or extending condition permits the 5' nuclease activity of the polymerase to cleave the annealed, labeled oligonucleotide and release labeled fragments; and
(c) detecting and/or measuring the release of labeled fragments to determine the presence or absence or amount of the target nucleic acid sequence in the sample.

11. The method of any one of the preceding claims, wherein the amplification is a polymerase chain reaction (PCR), wherein the hybridisation and the extension conditions are repeated at least once in a single round of thermal cycling if the hybridisation and the extension conditions are not the same conditions, wherein said single round of thermal cycling comprises temperatures for denaturing, hybridising, extending, hybridising and extending.

12. The method of any one of the preceding claims, wherein the amplification is an isothermal amplification.

## Patentansprüche

1. Verfahren für das Nachweisen oder Messen einer Zielnukleinsäuresequenz in einer Probe, wobei das Verfahren umfasst:
(a) Bereitstellen für ein Amplifikationsassay enthaltend die Probe:
(i) ein Paar von Amplifikationsprimern,
wobei der erste Amplifikationsprimer eine Sequenz enthält, die komplementär ist zu einer Region eines Strangs der Zielnukleinsäure und in der Lage ist, die Synthese des komplementären Strangs zu primen,
und wobei der zweite Primer eine Sequenz enthält, die komplementär ist zu einer Region des zweiten Strangs der Zielnukleinsäure und in der Lage ist, die Synthese eines dazu komplementären Strangs zu primen, dermaßen, dass nach der Amplifikation in der Anwesenheit der Zielnukleinsäure, der Abschnitt der Zielnukleinsäure zwischen den Amplifikationsprimern amplifiziert wird;
(ii) ein Helferoligonukleotid enthaltend eine Sequenz, die komplementär ist zu einer ersten Region der Zielnukleinsäure;
(iii) ein markiertes Oligonukleotid enthaltend eine Sequenz, die komplementär ist zu einer zweiten Region der Zielnukleinsäure,
wobei das Helferoligonukleotid und das markierte Oligonukleotid beide an den gleichen Strang der Zielnukleinsäure binden,
wobei die zweite Region sich neben dem 5'-Ende der ersten Region befindet oder teilweise die erste Region umfasst, und
wobei das Helferoligonukleotid und das markierte Oligonukleotid beide an die Zielnukleinsäureregion binden, welche sich zwischen den Bindungsregionen der Amplifikationsprimer befindet,
wobei,
falls die Zielnukleinsäure in der Probe vorliegt, das Helferoligonukleotid und das markierte Oligonukleotid dermaßen an die Zielnukleinsäure binden, dass das 3'-Ende des Helferoligonukleotids sich neben dem 5'-Ende des markierten Oligonukleotids befindet oder damit überlappt,
(b) Durchführen einer Amplifikationsreaktion unter Verwendung einer Nukleinsäurepolymerase mit 5'-Nukleaseaktivität unter Bedingungen, welche, falls die Zielnukleinsäure in der Probe vorliegt, die folgenden Schritte zulassen würde:
(i) Hybridisieren der Amplifikationsprimer, des Helferoligonukleotids und des markierten Oligonukleotids an die Zielnukleinsäure,
wobei die 5'-Nukleaseaktivität der Polymerase das gebundene, markierte Oligonukleotid auf eine polymerisationsunabhängige Art und Weise spaltet, um markierte Fragmente freizusetzen, welche nachweisbar sind; und
(ii) Erweitern der Amplifikationsprimer, wobei die Nukleinsäurepolymerase Primererweiterungsprodukte synthetisiert; und
(c) Nachweisen und/oder Messen der Freisetzung von markierten Fragmenten, um die Anwesenheit oder Abwesenheit oder Menge der Zielnukleinsäuresequenz in der Probe festzustellen.

2. Verfahren nach Anspruch 1, wobei das gebundene Helferoligonukleotid die Hybridisierung des markierten Oligonukleotids erleichtert und/oder die Spaltung des gebundenen, markierten Oligonukleotids verbessert, wobei die Hybridisierungsbedingung und/oder Erweiterungsbedingung es der 5'-Nukleaseaktivität der Polymerase erlaubt, das gebundene, markierte Oligonukleotid zu spalten und markierte Fragmente freizusetzen.

3. Verfahren nach Anspruch 1 oder Anspruch 2, wobei das Spalten des markierten Oligonukleotids während der Erweiterung der Amplifikation stattfindet.

4. Verfahren nach einem der Ansprüche 1 bis 3, wobei entweder:
(A) die Hybridisierung und Erweiterung unter den gleichen Bedingungen stattfinden; oder
(B) die Hybridisierungsbedingung und Erweiterungsbedingung wenigstens einmal in einer einzelnen Runde eines thermalen Cyclings wiederholt werden, wenn die Hybridisierungs- und Erweiterungsbedingungen nicht die gleichen Bedingungen sind.

5. Verfahren nach einem der Anspruch 1 bis 4, wobei entweder:
(A) das 3'-Ende des Helferoligonukleotids in dem gebundenen Duplex sich neben dem 5'-Ende eines gebundenen, markierten Oligonukleotids befindet, mit einem Abstand, welcher das Spalten des markierten Oligonukleotids in der Abwesenheit einer Nukleinsäurepolymerisation erlauben kann, wobei der Abstand zwischen dem 3'-Ende des Helferoligonukleotids im gebundenen Duplex und dem 5'-Ende eines gebundenen, markierten Oligonukleotid von 0 bis 10 Nukleotide ist; oder
(B) das 3'-Ende des Helferoligonukleotids in dem gebundenen Duplex mit dem 5'-Ende eines gebundenen, markierten Oligonukleotids überlappt, wobei die überlappende Region das Spalten des markierten Oligonukleotids in der Abwesenheit einer Nukleinsäurepolymerisation erlauben kann, wobei die überlappende Region zwischen dem 3'-Ende des Helferoligonukleotids in dem gebundenen Duplex und dem 5'-Ende eines gebundenen, markierten Oligonukleotids 1 bis 15 Nukleotide aufweist.

6. Verfahren nach einem der vorherigen Ansprüche, wobei das 3'-Ende des Helferoligonukleotids in dem gebundenen Duplex ein einzelnes Nukleotid am 3'-Terminus aufweist, welches zu der Zielnukleinsäuresequenz nicht komplementär ist.

7. Verfahren nach einem der vorherigen Ansprüche, wobei das markierte Oligonukleotid wenigstens eine Markierung umfasst, wobei das markierte Oligonukleotid erste und zweite Markierungen umfasst, wobei die erste Markierung von der zweiten Markierung durch eine nukleaseempfindliche Schnittstelle getrennt ist.

8. Verfahren nach einem der vorherigen Ansprüche, wobei das Helferoligonukleotid während der Amplifikation nicht erweitert wird.

9. Verfahren nach einem der vorherigen Ansprüche, wobei das markierte Oligonukleotid eine einzelsträngige, mit zwei Farbstoffen markierte TaqMan-Sonde ist oder eine Molecular-Beacon-Sonde ist oder einer der Stränge einer teilweise doppelsträngigen Sonde ist, wobei die teilweise doppelsträngige Sonde ein erstes Oligonukleotid umfasst, welches in der Lage ist, an das zweite Oligonukleotid zu hybridisieren, wobei jedes der ersten und zweiten Oligonukleotide einen Teil eines interaktiven Markerpaars umfasst.

10. Verfahren zum Überwachen einer Nukleinsäureamplifikation umfassend:
(a) Bereitstellen für ein Amplifikationsassay enthaltend die Probe:
(i) ein Paar von Amplifikationsprimern,
wobei der erste Amplifikationsprimer eine Sequenz enthält, die komplementär ist zu einer Region eines Strangs der Zielnukleinsäure und in der Lage ist, die Synthese des komplementären Strangs zu primen,
und wobei der zweite Primer eine Sequenz enthält, die komplementär ist zu einer Region des zweiten Strangs der Zielnukleinsäure und in der Lage ist, die Synthese eines dazu komplementären Strangs zu primen, dermaßen, dass nach der Amplifikation in der Anwesenheit der Zielnukleinsäure, der Abschnitt der Zielnukleinsäure zwischen den Amplifikationsprimern amplifiziert wird;
(ii) ein Satz von mehrfach markierten Oligonukleotiden, wobei jedes der markierten Oligonukleotide an den gleichen Strang der Zielnukleinsäure bindet,
wobei die gesamten markierten Oligonukleotide an die Zielnukleinsäureregion binden, welche sich zwischen den Bindungsregionen der Amplifikationsprimer befindet,
wobei,
falls die Zielnukleinsäure in der Probe vorliegt, die markierten Oligonukleotide dermaßen an die Zielnukleinsäure binden, dass das 3'-Ende eines des markierten Oligonukleotids sich neben dem 5'-Ende eines stromabwärts markierten Oligonukleotids befindet oder damit überlappt, eines der Amplifikationsprimer sich neben dem 5'-Ende eines stromabwärts markierten Oligonukleotids befindet oder damit überlappt;
(b) Durchführen einer Amplifikationsreaktion unter Verwendung einer Nukleinsäurepolymerase mit 5'-Nukleaseaktivität unter Bedingungen, welche, falls die Zielnukleinsäure in der Probe vorliegt, die folgenden Schritte zulassen würden:
(i) Hybridisieren der Amplifikationsprimer, markierten Oligonukleotide an die Zielnukleinsäure,
wobei das gebundene, stromaufwärts markierte Oligonukleotid auf eine polymerisationsunabhängige Art und Weise als Helfernukleotid wirkt, die Hybridisierung des stromabwärts markierten Oligonukleotids erleichtert und/oder das Spalten des gebundenen, markierten Oligonukleotids durch die 5'-Nukleaseaktivität der Polymerase erleichtert, um markierte Fragmente freizusetzen, welche nachweisbar sind; und
(ii) Erweitern der Amplifikationsprimer, wobei die Nukleinsäurepolymerase Primererweiterungsprodukte synthetisiert; und
wobei die Hybridisierungsbedingung und/oder Erweiterungsbedingung es der 5'-Nukleaseaktivität der Polymerase erlaubt, das gebundene, markierte Oligonukleotid zu spalten und markierte Fragmente freizusetzen; und
(c) Nachweisen und/oder Messen der Freisetzung von markierten Fragmenten, um die Anwesenheit oder Abwesenheit oder Menge der Zielnukleinsäuresequenz in der Probe festzustellen.

11. Verfahren nach einem der vorherigen Ansprüche, wobei die Amplifikation eine Polymerasekettenreaktion (engl. *polymerase chain reaction,* PCR) ist, wobei die Hybridisierungs- und Erweiterungsbedingungen wenigstens einmal in einer einzelnen Runde eines thermalen Cyclings wiederholt werden, falls die Hybridisierungs- und Erweiterungsbedingungen nicht die gleichen Bedingungen sind, wobei die einzelne Runde des thermalen Cyclings Temperaturen zum Denaturieren, Hybridisieren, Erweitern, Hybridisieren und Erweitern umfasst.

12. Verfahren nach einem der vorherigen Ansprüche, wobei die Amplifikation eine isothermale Amplifikation ist.

## Revendications

1. Procédé de détection ou de mesure d'une séquence d'acide nucléique cible dans un échantillon, ledit procédé comprenant :
(a) la mise à disposition, dans un essai d'amplification contenant ledit échantillon, de :
(i) une paire d'amorces d'amplification,
où la première amorce d'amplification contient une séquence complémentaire à une région d'un seul brin de l'acide nucléique cible et est capable d'amorcer la synthèse du brin complémentaire,
et où la seconde amorce contient une séquence complémentaire à une région du second brin de l'acide nucléique cible et est capable d'amorcer la synthèse d'un brin qui est complémentaire à celle-ci,
de sorte que, suite à une amplification en présence de l'acide nucléique cible, le segment d'acide nucléique cible entre les amorces d'amplification soit amplifié ;
(ii) un oligonucléotide auxiliaire contenant une séquence complémentaire à une première région de l'acide nucléique cible ;
(iii) un oligonucléotide marqué contenant une séquence complémentaire à une seconde région de l'acide nucléique cible ;
où l'oligonucléotide auxiliaire et l'oligonucléotide marqué s'annèlent tous deux au même brin de l'acide nucléique cible, où la seconde région est adjacente à l'extrémité 5' de la première région, ou comprend partiellement la première région, et
où l'oligonucléotide auxiliaire et l'oligonucléotide marqué s'annèlent tous deux au sein de la région de l'acide nucléique cible comprise entre les régions de liaison des amorces d'amplification,
dans lequel
si l'acide nucléique cible est présent dans l'échantillon, l'oligonucléotide auxiliaire et l'oligonucléotide marqué s'annèlent à l'acide nucléique cible de sorte que l'extrémité 3' de l'oligonucléotide auxiliaire soit adjacente à ou chevauche l'extrémité 5' de l'oligonucléotide marqué,
(b) la réalisation d'une réaction d'amplification en utilisant une polymérase d'acide nucléique ayant une activité nucléase 5' dans des conditions qui, si l'acide nucléique cible est présent dans l'échantillon, seraient permissives pour les étapes :
(i) d'hybridation des amorces d'amplification, de l'oligonucléotide auxiliaire et de l'oligonucléotide marqué à l'acide nucléique cible,
où l'activité nucléase 5' de la polymérase clive l'oligonucléotide marqué et annelé, de manière indépendante de la polymérisation, afin de libérer des fragments marqués qui sont détectables ; et
(ii) d'extension des amorces d'amplification, où la polymérase d'acide nucléique synthétise des produits d'extension d'amorce ; et
(c) la détection et/ou la mesure de la libération des fragments marqués afin de déterminer la présence ou l'absence ou la quantité de la séquence d'acide nucléique cible dans l'échantillon.

2. Procédé selon la revendication 1, dans lequel l'oligonucléotide auxiliaire annelé facilite l'hybridation de l'oligonucléotide marqué et/ou améliore le clivage de l'oligonucléotide marqué et annelé, où la condition d'hybridation et/ou condition d'extension permet à l'activité nucléase 5' de la polymérase de cliver l'oligonucléotide marqué et annelé et de libérer les fragments marqués.

3. Procédé selon la revendication 1 ou la revendication 2, dans lequel le clivage de l'oligonucléotide marqué se produit pendant l'extension de l'amplification.

4. Procédé selon l'une quelconque des revendications 1 à 3, dans lequel soit :
(A) l'hybridation et l'extension se produisent dans les mêmes conditions ; ou
(B) la condition d'hybridation et la condition d'extension sont répétées au moins une fois dans un seul tour de cycle thermique, si les conditions d'hybridation et d'extension ne sont pas les mêmes conditions.

5. Procédé selon l'une quelconque des revendications 1 à 4, dans lequel soit :
(A) l'extrémité 3' de l'oligonucléotide auxiliaire dans le duplex annelé est adjacente à l'extrémité 5' d'un oligonucléotide marqué et annelé, avec un espacement efficace pour permettre le clivage de l'oligonucléotide marqué en l'absence de polymérisation de l'acide nucléique, où ledit espacement entre l'extrémité 3' de l'oligonucléotide auxiliaire dans le duplex annelé et l'extrémité 5' d'un oligonucléotide marqué et annelé est compris entre 0 et 10 nucléotides ; ou
(B) l'extrémité 3' de l'oligonucléotide auxiliaire dans le duplex annelé chevauche l'extrémité 5' d'un oligonucléotide marqué et annelé, avec une région chevauchante efficace pour permettre le clivage de l'oligonucléotide marqué en l'absence de polymérisation de l'acide nucléique, où ladite région chevauchante entre l'extrémité 3' de l'oligonucléotide auxiliaire dans le duplex annelé et l'extrémité 5' d'un oligonucléotide marqué et annelé possède 1 à 15 nucléotides.

6. Procédé selon l'une quelconque des revendications précédentes, dans lequel l'extrémité 3' de l'oligonucléotide auxiliaire dans le duplex annelé possède un seul nucléotide à l'extrémité 3' non complémentaire à la séquence d'acide nucléique cible.

7. Procédé selon l'une quelconque des revendications précédentes, dans lequel l'oligonucléotide marqué comprend au moins un marqueur, où l'oligonucléotide marqué comprend un premier et un second marqueur, où le premier marqueur est séparé du second marqueur par un site de clivage sensible aux nucléases.

8. Procédé selon l'une quelconque des revendications précédentes, dans lequel l'oligonucléotide auxiliaire n'est pas étendu pendant l'amplification.

9. Procédé selon l'une quelconque des revendications précédentes, dans lequel ledit oligonucléotide marqué est une sonde Taqman simple brin marqué par un double colorant, ou est une sonde de balise moléculaire, ou est l'un des brins d'une sonde partiellement double brin, où ladite sonde partiellement double brin comprend un premier oligonucléotide qui est capable de s'hybrider à un second oligonucléotide, où chacun du premier et second oligonucléotide comprend un membre d'une paire de marqueurs interactive.

10. Procédé pour surveiller l'amplification d'un acide nucléique comprenant :
(a) la mise à disposition, dans un essai d'amplification contenant ledit échantillon, de :
(i) une paire d'amorces d'amplification,
où la première amorce d'amplification contient une séquence complémentaire à une région d'un seul brin de l'acide nucléique cible et est capable d'amorcer la synthèse du brin complémentaire,
et où la seconde amorce contient une séquence complémentaire à une région du second brin de l'acide nucléique cible et est capable d'amorcer la synthèse d'un brin qui est complémentaire à celle-ci,
de sorte que, suite à une amplification en présence de l'acide nucléique cible, le segment d'acide nucléique cible entre les amorces d'amplification soit amplifié ;
(ii) un ensemble de plusieurs oligonucléotides marqués, où chacun des oligonucléotides marqués s'annèle au même brin de l'acide nucléique cible, où tous les oligonucléotides marqués s'annèlent au sein de la région d'acide nucléique cible comprise entre les régions de liaison des amorces d'amplification,
dans lequel
si l'acide nucléique cible est présent dans l'échantillon, les oligonucléotides marqués s'annèlent à l'acide nucléique cible de sorte que l'extrémité 3' de l'un des oligonucléotides marqués soit adjacente à ou chevauche l'extrémité 5' d'un oligonucléotide marqué en aval, et l'une des amorces d'amplification est adjacente à ou chevauche l'extrémité 5' d'un oligonucléotide marqué en aval ;
(b) la réalisation d'une réaction d'amplification en utilisant une polymérase d'acide nucléique ayant une activité nucléase 5' dans des conditions qui, si l'acide nucléique cible est présent dans l'échantillon, seraient permissives pour les étapes :
(i) d'hybridation des amorces d'amplification et des oligonucléotides marqués à l'acide nucléique cible,
où l'oligonucléotide marqué en amont annelé agit comme un oligonucléotide auxiliaire, facilite l'hybridation de l'oligonucléotide marqué en aval et/ou améliore le clivage de l'oligonucléotide marqué et annelé par l'activité nucléase 5' de la polymérase, d'une manière indépendante de la polymérisation, afin de libérer des fragments marqués qui sont détectables ; et
(ii) d'extension des amorces d'amplification, où la polymérase d'acide nucléique synthétise des produits d'extension d'amorce,
où la condition d'hybridation et/ou la condition d'extension permet à l'activité nucléase 5' de la polymérase de cliver l'oligonucléotide marqué et annelé et de libérer les fragments marqués ; et
(c) la détection et/ou la mesure de la libération des fragments marqués afin de déterminer la présence ou l'absence ou la quantité de la séquence d'acide nucléique cible dans l'échantillon.

11. Procédé selon l'une quelconque des revendications précédentes, dans lequel l'amplification est une réaction en chaîne par polymérase (PCR), où les conditions d'hybridation et d'extension sont répétées au moins une fois en un seul tour de cycle thermique si les conditions d'hybridation et d'extension ne sont pas les mêmes conditions, où ledit un seul tour de cycle thermique comprend des températures de dénaturation, d'hybridation, d'extension, d'hybridation et d'extension.

12. Procédé selon l'une quelconque des revendications précédentes, dans lequel l'amplification est une amplification isotherme.


### SEQUENCE LISTING

<110> Oxitec Ltd
<120> Enhanced Taqman probe-based amplification
<130> 489.101397/03
<160> 13
<170> PatentIn version 3.3
<210> 1
   <211> 22
   <212> DNA
   <213> Artificial
<220>
   <223> Forward amplification primer
<400> 1
   cctttactta ctacacctca ga 22
<210> 2
   <211> 23
   <212> DNA
   <213> Artificial
<220>
   <223> Reverse amplification primer
<400> 2
   gcctcaattc ttaccatcca caa 23
<210> 3
   <211> 30
   <212> DNA
   <213> Artificial
<220>
   <223> Labelled oligonucleotide probe
<400> 3
   ctcccatcag tttgaacagt tgtctgggag 30
<210> 4
   <211> 20
   <212> DNA
   <213> Artificial
<220>
   <223> Helper oligonucleotide
<400> 4
   tgaaatctcg atggagtggg 20
<210> 5
   <211> 34
   <212> DNA
   <213> Artificial
<220>
   <223> Forward amplification primer
<400> 5
   gtcacatttt cattattttt attataaggc ctgc 34
<210> 6
   <211> 28
   <212> DNA
   <213> Artificial
<220>
   <223> Reverse amplification primer
<400> 6
   ctctattgtt ggatcatatt cgtccaca 28
<210> 7
   <211> 25
   <212> DNA
   <213> Artificial
<220>
   <223> Helper oligonucleotide
<400> 7
   gtcaaggcac tcttgcctac gccaa 25
<210> 8
   <211> 21
   <212> DNA
   <213> Artificial
<220>
   <223> oligonucleotide probe
<400> 8
   ccagctccaa ctaccacaag t 21
<210> 9
   <211> 27
   <212> DNA
   <213> Artificial
<220>
   <223> Forward amplification primer
<400> 9
   gtggagacga gagtaagtaa aactaca 27
<210> 10
   <211> 29
   <212> DNA
   <213> Artificial
<220>
   <223> Reverse amplification primer
<400> 10
   ctcctgttaa attatagttt acactgaca 29
<210> 11
   <211> 33
   <212> DNA
   <213> Artificial
<220>
   <223> Helper oligonucleotide
<400> 11
   gatcctgaaa ctgaattttc tatataaaca aac 33
<210> 12
   <211> 27
   <212> DNA
   <213> Artificial
<220>
   <223> oligonucleotide probe
<400> 12
   aacagatgct ctgagaaagg cattaga 27
<210> 13
   <211> 15
   <212> DNA
   <213> Artificial
<220>
   <223> oligonucleotide probe
<400> 13
   ctcagagcat ctgtt 15